# EUROPEAN PATENT APPLICATION

(11) **EP 2 719 375 A1**
(43) Date of publication of application: **16.04.2014**
(21) Application number: 12188038.9
(22) Date of filing: 10.10.2012
(51) Int. Cl.: A61K 9/127, A61K 31/20, A61K 31/232, A61K 31/35, A61K 47/00, A61P 35/00

(54) **Cannabinoids for the treatment of cancers dependent on hedgehog mechanisms**

(71) Applicant: Max-Planck-Gesellschaft zur Förderung der Wissenschaften e.V., 80539 München (DE)
(72) Inventor: Eaton, Suzanne, 01309 Dresden (DE); Khaliullina, Helena, 01277 Dresden (DE)
(74) Representative: Vossius & Partner

(57) **Abstract**

The present invention relates to a pharmaceutical composition comprising one or more endocannabinoids, cannabinoids and/or modified versions thereof complexed with one or more lipoproteins, wherein said one or more endocannabinoids are selected from N-acylethanolamides, N-acyl dopamines, 2-acyl glycerols and 2-glyceryl ethers and wherein said one or more cannabinoids are selected from cannabinols, cannabidiols and tetrahydrocannabinols. Further, the invention relates to the use of said pharmaceutical composition in the treatment of a tumour in a mammal. Also, the invention relates to a method for modulating the activity of the hedgehog signaling pathway in a mammalian cell and the use of one or more endocannabinoids, cannabinoids and modified versions thereof in the differentiation of embryonic stem cells, progenitor cells and/or induced stem cells.

## Description

The present invention relates to a pharmaceutical composition comprising one or more endocannabinoids, cannabinoids and/or modified versions thereof complexed with one or more lipoproteins, wherein said one or more endocannabinoids are selected from N-acylethanolamides, N-acyl dopamines, 2-acyl glycerols and 2-glyceryl ethers and wherein said one or more cannabinoids are selected from cannabinols, cannabidiols and tetrahydrocannabinols. Further, the invention relates to the use of said pharmaceutical composition in the treatment of a tumour in a mammal. Also, the invention relates to a method for modulating the activity of the Hedgehog signaling pathway in a mammalian cell and the use of one or more endocannabinoids, cannabinoids and modified versions thereof in the differentiation of embryonic stem cells, progenitor cells and/or induced stem cells.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Historically, tumors have been categorized based on their tissue of origin and histopathology, and drug therapies have been chosen empirically based on these categories. Recent progress in our understanding of the different signaling pathways that can drive tumorigenesis has allowed the development of specific pathway inhibitors, but it is becoming increasingly clear that even tumors from the same cell type of origin can be very heterogeneous in terms of the molecular pathways that drive their growth. Thus, there has been a shift away from defining cancers based on their tissues of origin, and towards a diagnosis based on molecular alterations in specific pathways. This will become the standard more generally as the fall in sequencing costs make analysis of individual patient tumors feasible (Chin et al., Nature Medicine, 2011. 17(3): p. 297-303; Stricker et al., Seminars in Oncology, 2011. 38(2): p. 173-85).

The Hedgehog (Hh) pathway has been implicated in growth and survival of many types of tumors, but with different frequencies depending on the type of tumor. For example, 95% of basal cell carcinomas have mutations in the Hedgehog pathway (Weiss, G.J. and R.L. Korn, Cancer, (2012), doi: 10.1002/cncnr.27532 (epub ahead of print)). In contrast, Hedgehog pathway components are mutated in only about 25% of medulloblastomas. This does not appear to correlate with any particular histological grouping of medulloblastomas, emphasizing the importance of molecular rather than morphological diagnosis (Pugh et al., Nature, 488(7409): p. 106-10 (2012); Lau et al., Child's nervous system : ChNS : official journal of the International Society for Pediatric Neurosurgery, 28(4): p. 521-32 (2012)).

As the molecular characterization of individual patient tumors becomes more common, only drugs for which the molecular and cellular mechanisms of action are understood will be able to exploit this new information to correctly match a specific drug with the patient subgroup in which it is likely to be active

Hedgehog proteins control growth and development throughout the animal kingdom, and have active roles in adult life in a variety of stem cell types. Hedgehog proteins signal by repressing the activity of the 12-pass transmembrane protein Patched (Ptc). When Hh is absent, Ptc catalytically represses the activity of the 7-pass transmembrane protein Smoothened (Smo). Smo regulates the processing and localization of the Gli family of transcription factors, modulating the balance of transcriptional activation and transcriptional repression of different target genes (Varjosalo, M. and J. Taipale, Genes & Development, 22(18): p. 2454-72 (2008)).

Ptc is a member of the RND family of proton-driven transporters, which includes proteins that flux small hydrophobic molecules across cell membranes. It regulates intracellular lipoprotein trafficking and the efflux of lipoprotein lipids from endosomes (Khaliullina et al., Development, 136(24): p. 4111-21 (2009)). It is thought to control Smo activity by regulating access of one or more lipidic Smo inhibitors (Taipale et al., Nature, 418(6900): p. 892-7 (2002)). A variety of exogenous molecules have been shown to bind to and inhibit Smo (Frank-Kamenetsky et al., Journal of Biology, 1(2): p. 10 (2002)). Some of these appear to bind to different sites on Smo and to block its activity at separable steps, suggesting that Smo, like other GPCR's (G-protein coupled receptor) may interact with and respond to multiple ligands. The first of these was the steroidal alkaloid cyclopamine, derived from *Veratrum californicum,* a wild California grass (Chen et al., Genes & Development, 16(21): p. 2743-8 (2002)). Improved derivatives of cyclopamine have given promising results in Phase I and II clinical trials, as have several other artificial compounds from small molecule screens. These include GDC-0449 from Genentech/Curis/Roche, LDE-225 and LEQ-506 from Novartis, BMS-833923 from Bristol-Myers-Squibb, XL139 from Exelixis, IPI-926 from Infinity, PF-0449913 from Pfizer, and TAK-441 from Milenium (Tremblay et al., Current opinion in chemical biology, 14(3): p. 428-35 (2010); Lin, T.L. and W. Matsui, OncoTargets and Therapy, 5: p. 47-58 (2012)). While the initial response to treatment has been promising, tumors later developed resistance, in some cases by the occurrence of second mutations in Smoothened (Metcalfe, C. and F.J. de Sauvage, Cancer Research, 71(15): p. 5057-61 (2011)).

Endogenous ligands for Smo are only beginning to be identified. These could in principle themselves be important therapeutic molecules, or suggest novel scaffolds for the development of even more potent compounds. In mammals, vitamin D3 was shown to bind Smo at the same site as cyclopamine, and to inhibit Hh signaling in tissue culture and *in vivo* (Bijlsma, M.F., et al., PLoS biology, 2006. 4(8): p. e232).

Despite the progress in elucidating the molecular mechanisms underlying cancer, there is still a need for providing appropriate approaches for combating cancer. The technical problem underlying the present invention was thus to identify alternative and/or improved means and methods to treat tumours.

The solution to this technical problem is achieved by providing the embodiments characterized in the claims.

Accordingly, the present invention provides a pharmaceutical composition comprising one or more endocannabinoids, cannabinoids and/or modified versions thereof complexed with one or more lipoproteins, wherein said one or more endocannabinoids are selected from N-acylethanolamides, N-acyl dopamines, 2-acyl glycerols and 2-glyceryl ethers and wherein said one or more cannabinoids are selected from cannabinols, cannabidiols and tetrahydrocannabinols.

The term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. As the endocannabinoids, cannabinoids and/or modified versions thereof recited herein above are complexed with one or more lipoproteins, said complex dictates the form of the pharmaceutical composition and the mode of administration. For example, said complex cannot not be formulated as a solid and given orally as a tablet. Thus, only formulations are to be chosen which do not negatively affect, e.g. destroy, the integrity of the lipoprotein-endocannabinoid complex. Preferably, said complex is in liquid form such as a solution or an aerosol. Further, preferred is the administration by infusion. The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, etc. Again, the endocannabinoids, cannabinoids and/or modified versions thereof recited herein above are complexed with one or more lipoproteins, said complex dictates which pharmaceutical carriers can be used and which not. Thus, only those further constituents, e.g. pharmaceutically acceptable carriers, are to be chosen which do not negatively affect, e.g. destroy, the integrity of the lipoprotein-endocannabinoid complex. For example, the use of DMSO and organic solvents will destroy a lipoprotein-endocannabinoid complex. Compositions comprising such carriers can be formulated by well-known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgment of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day. The length of treatment needed to observe changes and the interval following treatment for responses to occur vary depending on the desired effect. The particular amounts may be determined by conventional tests which are well known to the person skilled in the art.

Administration of the pharmaceutical compositions may be effected by different ways depending on the formulation and the constituents of said pharmaceutical compositions (see above), e.g., by topical, oral, rectal, intravenous, intraperitoneal, subcutaneous, intramuscular, intradermal, intranasal or intrabronchial administration, and depends on the tumour to be treated. As evident from the above, systemic and localized administration of the pharmaceutical composition is envisaged in accordance with the invention.

The term "endocannabinoids" is well known in the art. Initially, it referred to molecules that activate the cannabinoid receptors within the body (Maccarrone et al., Annu. Rev. Nutr. 30:423-440 (2010); Katona and Freund, Annu. Rev. Neurosci. 35:529-558 (2012)). 2-arachidonyl glycerol (2AG), 2-arachidonyl glyceryl ether (2AGE), arachidonyl dopamine, and arachidonyl ethanolamide (anandamide) were the first molecules identified with this capability. Since then, structurally related endogenous molecules have been identified that share similar structural features, but that display weak or no activity towards the cannabinoid receptors, but are also termed endocannabinoids. In many cases, their relevant *in vivo* receptors have not been identified. Examples of these endocannabinoid lipids include 2-acyl glycerols, alkyl or alkenyl glyceryl ethers, acyl dopamines and N-acylethanolamides that contain alternative fatty acid or alcohol moieties, as well as other fatty acid amides containing different head groups. These include N-acylserines as well as many other N-acylated amino acids. The known cannabinoid receptor agonists are neuromodulatory and affect short-term memory, appetite, stress response, anxiety, immune function and analgesia. Biological activities of other endocannabinoid family lipids are less well defined but include vasodilation (arachidonyl serine), and anti-inflammation (palmitoyl ethanolamide acting as a ligand for PPARalpha).

The endocannabinoids referred to herein are abbreviated using standard abbreviations well known in the art. Accordingly, the term "(x:y)" following the class name of a given endocannabinoid, is used to describe the total number of carbons (x) and the number of double bonds (y) of the fatty acid or alcohol moiety "R" (also referred to as lipid number nomenclature). For example, N-acylethanolamide (18:2) relates to an endocannabinoid from the N-acylethanolamide class having an 18 carbon fatty acid moiety with two double bonds. As such, if "y" ≥ 1, then an unsaturated fatty acid or alcohol moiety is part of the given endocannabinoid. "y" = 0 refers to a saturated fatty acid or alcohol.

As is known in the art, the above described nomenclature can further be refined in the case of unsaturated fatty acid or alcohol moieties by referring to the position of the double bond(s) as "Δ^{X}", where the double bond is located at the x^{th} carbon-carbon bond, counting from the carbonyl atom (designated as "Δ") toward the terminal methyl carbon. Further, it can be indicated whether the double bond is in cis- or trans-configuration. For example, N-acylethanolamide (18:2) cis-Δ⁹, Δ¹² refers to an N-acylethanolamide having an 18 carbon fatty acid moiety which has a double bond at the 9^{th} carbon and 12^{th} carbon atom from the carbonyl atom, wherein both double bonds are in cis-configuration. In trivial nomenclature, a corresponding fatty acid is called linoleic acid and, thus, the endocannabinoid can also be termed linoleylethanolamide.

Accordingly, and if not otherwise specified, when only using the "(x:y)" nomenclature for an unsaturated fatty acid or alcohol moiety, a corresponding endocannabinoid encompasses all possible cis- or trans-configurations of the double bond(s) and the double bond(s) can be situated at any carbon atom position. Preferably, in the case of more than one double bond, each carbon double bonds is followed by three carbon atoms. Exemplary, double bond positions are Δ⁵, Δ⁸, Δ⁹, Δ¹¹ Δ¹² and/or Δ¹⁴.

Also envisaged are modifications of the fatty acid or alcohol moiety. It is understood that any modification effected must not interfere with the pharmaceutical activity of the endocannabinoids (as laid out herein below) to an extent that abolishes said function. Preferably, a correspondingly modified endocannabinoid maintains said activity to 100% or at least to (for each value) 98%, 96, 94, 92, 90, 85, 80, 75, 70, 65, 60, 55, 50, 45, 40, 35, 30 or 25% in comparison to its unmodified counterpart.

The endocannabinoid class of "N-acylethanolamides" is well known in the art (Maccarrone et al., Annu. Rev. Nutr. 2010, 30:423-440; Katona and Freund, Annu. Rev. Neurosci. 2012, 35:529-558). Briefly, an N-acylethanolamide is made up of ethanolamine that is amide linked to a fatty acid moiety. The structural formula is depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl. In other terms, R is the aliphatic tail of a given fatty acid moiety that was amide linked to said ethanolamine.
Preferably, "R" is C₁₆-C₂₀ alkyl or C₁₆-C₂₀ alkenyl, such as C₁₆, C₁₇, C₁₈, C₁₉ or C₂₀ alkyl or alkenyl. Alkenyl is preferably a group having one, two, three or four double bonds. Preferably, the N-acylethanolamides are selected from the group consisting of N-acylethanolamides (16:0), (16:1) (more preferred (16:1) cis-Δ⁹), (18:2), (20:3) and (20:4). Most preferred are N-acylethanolamides (18:2) cis-Δ⁹, Δ¹² and (20:4) cis-Δ⁵, Δ⁸, Δ¹¹ Δ¹⁴.
N-acylethanolamides can be prepared synthetically, e.g., by heating esters with ethanolamine (Phillips, A, Journal of the American Chemical Society 73: 5557 (1951)). Also, N-acylethanolamides can be obtained commercially, e.g., from Avanti Polar Lipids, Tocris Bioscience, Biomol, Cayman Chemicals, Sigma-Aldrich or Fluka.

The generic structural formula for N-acylethanolamides is as follows:

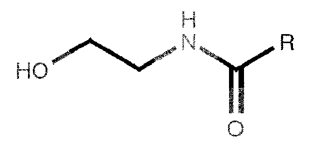

The endocannabinoid class of "N-acyl dopamines" is also well known in the art (Van der Stelt and Di Marzo, Eur. J. Biochem. 271, 1827-1834 (2004); Chu et al., The Journal of Biological Chemistry, 278, 16, pp. 13633-13639 (2003)). Briefly, an N-acyl dopamine is made up of dopamine that is amide linked to a fatty acid moiety. The structural formula is depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl. In other terms, R is the aliphatic tail of a given fatty acid moiety that was amide linked to said dopamine.
Preferably, "R" is C₁₆-C₁₈ alkyl or C₁₆-C₁₈ alkenyl, such as C₁₆, C₁₇ or C₁₈ alkyl or alkenyl. Also preferred, alkenyl is a group having one, two, three or four double bonds. More preferred, "R" is C₁₆-C₁₈ alkyl, such as in the case of N-acyl dopamine (16:0) and (18:0).
N-acyl dopamines can be obtained commercially, e.g., from Avanti Polar Lipids, Tocris Bioscience, Biomol, Cayman Chemicals, Sigma-Aldrich or Fluka.

The generic structural formula for N-acyl dopamines is as follows:

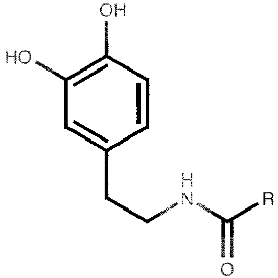

The endocannabinoid class of "2-acyl-glycerols" is also well known in the art (Maccarrone et al., Annu. Rev. Nutr. 30:423-440 (2010); Katona and Freund, Annu. Rev. Neurosci. 35:529-558 (2012)). Briefly, a 2-acyl-glycerol is made up of glycerol that is ester linked to a fatty acid moiety. The structural formula is depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl. Also preferred, "R" is C₁₆-C₂₀ alkyl or C₁₆-C₂₀ alkenyl, such as C₁₆, C₁₇, C₁₈, C₁₉ or C₂₀ alkyl or alkenyl. Alkenyl is preferably a group having one, two, three or four double bonds. Preferably, the 2-acyl-glycerol is 2-arachidonyl glycerol.
2-acyl-glycerols can be obtained commercially, e.g., from Avanti Polar Lipids, Tocris Bioscience, Biomol, Cayman Chemicals, Sigma-Aldrich, or Fluka.

The generic structural formula for 2-acyl-glycerols is as follows:

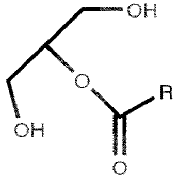

The endocannabinoid class of "2-glyceryl-ethers" is also well known in the art (Hanus et al., PNAS, vol. 98, no. 7, 3662-3665 (2001)). Briefly, a 2-glyceryl-ether is made up of glycerol that is ether linked at position 2 to a fatty alcohol moiety, which can be either saturated or unsaturated. The structural formula is depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl.
Also preferred, "R" is C₁₄-C₂₄ alkyl or C₁₄-C₂₄ alkenyl, such as C₁₆, C₁₇, C₁₈, C₁₉, C₂₀, C₂₁, C₂₂, C₂₃ or C₂₄ alkyl or alkenyl. Alkenyl is preferably a group having one, two, three or four double bonds. Preferably, the 2-glyceryl-ether is selected from the group consisting of 2-arachidonyl glyceryl ether (2-glyceryl ether (20:4)), 2-glyceryl ether (16:0), 2-glyceryl ether (16:1) and 2-glyceryl ether (18:1), and most preferred 2-arachidonyl glyceryl ether (2-glyceryl ether (20:4)).
2-glyceryl-ethers can be obtained commercially, e.g., from Avanti Polar Lipids, Tocris Bioscience, Biomol, Cayman Chemicals, Sigma-Aldrich, or Fluka.

The generic structural formula for 2-glyceryl-ethers is as follows:

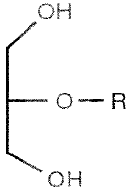

In accordance with the invention, the term "cannabinoid" is to refer to phytocannabinoids and synthetic cannabinoids. Phytocannabinoids are, e.g., tricyclic terpenoid compounds produced by *Cannabis sativa.* They are divided into different classes based on small differences in the ring structures. Different cannabinoid species in the same class are distinguished by various lengths of the C-3 side chain attached to the aromatic ring. They are known to be agonists for cannabinoid receptors CB1 and CB2 (Mechoulam, R., British Journal of Pharmacology, 146, 913-915 (2005)).

The cannabinoid class of "cannabinols" is represented by the structural formula depicted below, wherein "R" at the C-3 position is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl.
Also preferred, "R" is C₁-C₅ alkyl or alkenyl, such as C₁, C₂, C₃, C₄ or C₅ alkyl or alkenyl. Alkenyl is preferably a group having one, two or three double bonds.

Cannabinols can be obtained commercially, e.g., from Avanti Polar Lipids, Tocris Bioscience, Biomol, Cayman Chemicals, Sigma-Aldrich or Fluka.

The generic structural formula for cannabinols is as follows:

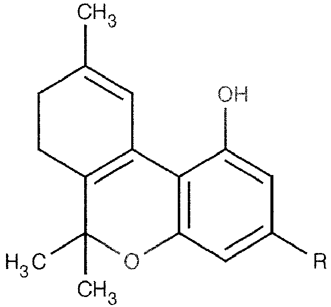

The cannabinoid class of "cannabidiols" is also well known in the art (Mechoulam, R., British Journal of Pharmacology (2005), 146, 913-915; Thakur et al., Life Sciences, 78, 454-466 (2005)) and is represented by the structural formula depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl.
Also preferred, "R" is C₁-C₅ alkyl or alkenyl, such as C₁, C₂, C₃, C₄ or C₅ alkyl or alkenyl, more preferred "R" is C₅ alkyl or alkenyl. Alkenyl is preferably a group having one, two or three double bonds
Cannabidiols can be obtained commercially, e.g., from Avanti Polar Lipids, Tocris Bioscience, Biomol, Cayman Chemicals, Sigma-Aldrich or Fluka.

The generic structural formula of cannabidiols is as follows:

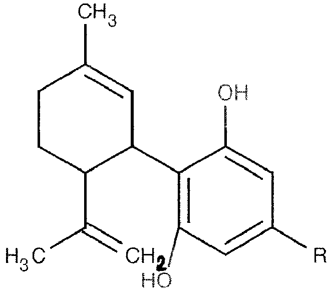

The cannabinoid class of "tetrahydrocannabinols" is also well known in the art (Mechoulam, R., British Journal of Pharmacology, 146, 913-915 (2005); Thakur et al., Life Sciences, 78, 454-466 (2005)). Briefly, a tetrahydrocannabinol has the general structure depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl.
Also preferred, "R" is C₁-C₅ alkyl or alkenyl, such as C₁, C₂, C₃, C₄ or C₅ alkyl or alkenyl.. The double bond in the first ring can be either in the Δ⁸ (as depicted) or Δ⁹ position.
Tetrahydrocannabinols can be prepared, e.g. by extraction from a cannabis plant. Also, tetrahydrocannabinols can be obtained commercially, e.g., from Tocris Bioscience, Biomol, Cayman Chemicals, Sigma-Aldrich or Fluka.

The generic structural formula of tetrahydrocannabinols is as follows:

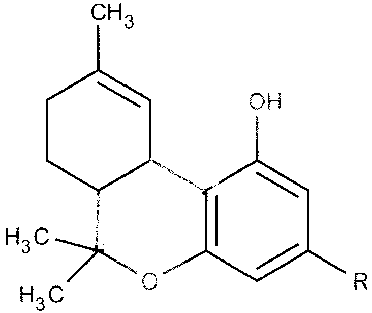

The same nomenclature as described herein above for the endocannabinoids also applies *mutatis mutandis* to cannabinoids.

The term "modified versions thereof' in accordance with the present invention refers to versions of the endocannabinoids or cannabinoids that are modified to achieve i) modified spectrum of activity, organ specificity, and/or ii) improved potency, and/or iii) decreased toxicity (improved therapeutic index), and/or iv) decreased side effects, and/or v) modified onset of therapeutic action, duration of effect, and/or vi) modified pharmacokinetic parameters (resorption, distribution, metabolism and excretion), and/or vii) modified physico-chemical parameters (solubility, hygroscopicity, color, taste, odor, stability, state), and/or viii) improved general specificity, organ/tissue specificity, and/or ix) optimised application form and route by (a) esterification of carboxyl groups, or (b) esterification of hydroxyl groups with carboxylic acids, or (c) esterification of hydroxyl groups to, e.g. phosphates, pyrophosphates or sulfates or hemi-succinates, or (d) formation of pharmaceutically acceptable salts, or (e) formation of pharmaceutically acceptable complexes, or (f) synthesis of pharmacologically active polymers, or (g) introduction of hydrophilic moieties, or (h) introduction/exchange of substituents on aromates or side chains, change of substituent pattern, or (i) modification by introduction of isosteric or bioisosteric moieties, or (j) synthesis of homologous compounds, or (k) introduction of branched side chains, or (k) conversion of alkyl substituents to cyclic analogues, or (I) derivatisation of hydroxyl groups to ketales, acetales, or (m) N-acetylation to amides, phenylcarbamates, or (n) synthesis of Mannich bases, imines, or (o) transformation of ketones or aldehydes to Schiffs bases, oximes, acetales, ketales, enolesters, oxazolidines, thiazolidines; or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The term "lipoprotein" is used herein according to its well-known meaning in the art. Briefly, a lipoprotein is a biochemical assembly of proteins and lipids bound to the proteins. They are generally organized as a polar lipid monolayer surrounding a neutral lipid core assembled onto an apolipoprotein scaffold (Breslow et al., Ann. Rev. Biochem., 54:699-727 (1985)). Lipoproteins may be classified by density and diameter, and by the specific apolipoprotein that acts as a scaffold. Chylomicrons are lipoproteins which are produced in the intestine and are scaffolded by apoB48. They have a density of below 0.95 g/ml and a diameter of 100 to 1000 nm. Very-low-density lipoproteins (VLDL), which are scaffolded by ApoB100, are produced in the liver and have a density of 0.95 g/ml to 1.006 g/mi and a diameter of 30 to 80 nm. Intermediate-density lipoproteins (IDL) are derived from VLDL in circulation as triglycerides in the neutral lipid core are hydrolysed to fatty acids and glycerol. They have a density of 1.006 g/ml to 1.019 g/ml and a diameter of 25 to 50 nm. Further triglyceride hydrolysis produces low-density lipoproteins (LDL), which have a density of 1.019 g/ml to 1.063 g/ml and a diameter of 25 to 50 nm. High-density lipoproteins (HDL) are scaffolded by completely different apolipoprotein moieties, ApoA and ApoE. These apolipoproteins are secreted in non-lipidated forms by the liver and other tissues, and acquire their lipid cargo by efflux of lipids from cells in the periphery. They have a density of more than 1.063 g/ml to 1.210 g/ml and a diameter of 25 to 50 nm. Accordingly, the lipoproteins according to the invention encompass chylomicrons, VLDL, IDL, LDL and HDL. In other words, and independent from their designation, lipoproteins can have a density of below 0.95 g/ml up to 1.210 g/ml and a diameter of about 5 to about 1000. Preferably, VLDLs, IDLs, LDLs and/or HDLs are use in accordance with the invention. Any combination of said preferred lipoproteins can be used, i.e. a mixture of different lipoproteins can be employed. The term "about" as used herein means a deviation of up to ± 10% such as 7.5%, 5% or 2.5%.

The term "complexed" in the context of lipoproteins and endocannabinoids means that the endocannabinoids become incorporated into the outer polar lipid monolayer of the lipoprotein. This is accomplished by adding a solution of one or more endocannabinoids described herein to an aqueous solution containing lipoproteins. The endocannabinoid(s) partition into the outer polar lipid monolayer of the lipoprotein because they have similar hydrophobicities.

The pharmaceutical composition may comprise only one kind of endocannabinoid and/or cannabinoid species or may comprise multiple endocannabinoid and/or cannabinoid species. For example, at least (for each value) 2 such as 3, 4, 5, 6, 7 or 8, 9, 10 or more endocannabinoids and/or cannabinoids may be comprised in the pharmaceutical composition in accordance with the invention. It is understood that the endocannabinoids and cannabinoid species may originate from one or more of the above described classes.

Equally, the pharmaceutical composition is not limited to comprise one kind of lipoprotein species, although this is explicitly envisioned, but may comprise multiple lipoprotein species. For example, at least (for each value) 2 such as 3, 4, 5, 6, 7 or 8, 9, 10 or more different lipoproteins species may be comprised in the pharmaceutical composition in accordance with the invention.

The inventors surprisingly found a class of molecules with activity against the Hedgehog pathway that is structurally distinct from currently known inhibitors: the endocannabinoids and cannabinoids. Without being bound to a specific scientific theory, and without limitation, there is the possibility that these inhibitors act at the level of Smo or upstream thereof. The present data shows that Patched-dependent intracellular trafficking of lipoproteins complexed with endocannabinoids and cannabinoids is important for pathway repression that can generally be exploited for pharmaceutical purposes.

Even more surprising, it could be shown that not all endocannabinoids have the same hedgehog antagonist potency and, more important, some even act as agonists. This is a finding with far-reaching consequences as regards tumour treatment when considering using endocannabinoids and/or cannabinoids. Prior to the invention, a certain anti-proliferative and/or apoptotic activity of some endocannabinoids was shown in tumour cells of differing tissue origin and it was speculated that they act through the known cannabinoid receptors CB1 and CB2 as well as the vanilloid receptor VR1. Thus, the state of the art teaches that tumours originating from one kind of tissue can be treated by endocannabinoids. However, as shown by the present invention, a corresponding treatment approach is not warranted to be effective and may have severe implications for a patient, i.e. if an endocannabinoid that is an Hh-agonist is chosen for the treatment of a hedgehog signaling-dependent tumor. Only on the basis of the present invention, a tumour patient can be sure to be treated purposefully with endocannabinoids: prior to the treatment one will now check whether the tumour is a hedgehog-signaling-dependent tumour (e.g., by analyzing a tumour tissue sample), in which case a treatment with endocannabinoids provides for improved results due to their specific Hh-antagonistic nature in comparison to a different, non-hedgehog signaling dependent tumour, albeit of the same tissue. Further, specific, highly potent endocannabinoids can be chosen and the selection of an endocannabinoid with anti-tumour activity in a non-hedgehog signaling-dependent tumour type, but which is an agonist of hedgehog signaling, can be avoided. Patients can now be treated which exhibit or are at risk to develop a resistance to other hedgehog signaling pathway inhibitors. In other words, the present invention enables a medical practitioner to select the best therapy for a subject afflicted with a hedgehog signaling-dependent tumour when using endocannabinoids or cannabinoids as treatment.

In summary, the finding that different classes of endocannabinoids modulate the Hedgehog pathway, and that endocannabinoid efficacy is increased when they are delivered to cells complexed with lipoproteins (the delivery mechanism that is relevant to Hedgehog signaling *in vivo*) is a powerful targeted tool to treat hedgehog signaling-dependent tumours. Thus, the invention is a further step towards warranting a more personalized tumour treatment.

The limitations, combinations and preferred embodiments described herein above for the first embodiment in relation to endocannabinoids, cannabinoids and/or modified versions thereof apply *mutatis mutandis* to the further embodiments described herein below if not expressly stated otherwise.

In a further embodiment, the invention relates to a pharmaceutical composition comprising one or more endocannabinoids, cannabinoids and/or modified versions thereof, wherein said one or more endocannabinoids are selected from N-acylethanolamides with the exception of N-acylethanolamide (16:0), N-acylethanolamide (18:1) and N-acylethanolamide (20:4); n-acyl-dopamines with the exception of N-acyl dopamine (16:0); 2-acyl glycerols with the exception of 2-acyl glycerol (20:4); and 2-glyceryl ethers with the exception of 2-glyceryl ether (20:4); and wherein said one or more cannabinoids are selected from cannabinols with the exception of cannabinol; cannabidiols with the exception of cannabidiol and tetrahydrocannabinols with the exception of tetrahydrocannabinol.

The same definitions as for the main embodiment also apply to this embodiment *mutatis mutandis* unless expressly mentioned otherwise. As the endocannabinoids, cannabinoids and/or modified versions thereof are not complexed with one or more lipoproteins in this embodiment, more options relating to the formulation of the pharmaceutical composition and its mode of administration are available. For example, the composition may additionally be in solid or gaseous form and may be, inter alia, in the form of (a) powder(s) or (a) tablet(s). Also, pharmaceutical carriers such as organic solvents or DMSO can be employed in the absence of said one or more lipoproteins in the pharmaceutical composition.

The term "with the exception of" in the context of endocannabinoid or cannabinoid classes is meant to specifically exclude a given endocannabinoid or cannabinoid species from a given endocannabinoid class that is recited herein above.

In this embodiment, the N-acylethanolamides preferably have a fatty acid moiety consisting of a 16 to 20 carbon atom backbone and between 0 and 4 double bonds, excluding N-acylethanolamide (16:0) (palmitic acid), N-acylethanolamide (20:4) (arachidonic acid) and N-acylethanolamide (18:1) (oleic acid). In other terms, "R" is C₁₆-C₂₀ alkyl or C₁₆-C₂₀ alkenyl, wherein alkenyl is preferably a group having one, two, three or four double bonds, excluding N-acylethanolamide (16:0) (palmitic acid), N-acylethanolamide (20:4) (arachidonic acid) and N-acylethanolamide (18: 1) (oleic acid).

In this embodiment, the n-acyl-dopamines preferably have a fatty acid moiety consisting of a 17 or 18 carbon atom backbone and no double bonds. In other terms, "R" is C₁₇ or C₂₀ alkyl.

In this embodiment, the 2-acyl-glycerols preferably have a fatty acid moiety consisting of a 16 and 20 carbon atom backbone, such as 16, 17, 18, 19 or 20 carbon atoms, and between 0 to 4 double bonds, such as 0, 1, 3, or 4 double bonds, excluding 2-acyl-glycerol (20:4) (2-arachidonyl glycerol).

In other terms, "R" is C₁₆-C₂₀ alkyl or C₁₆-C₂₀ alkenyl, wherein alkenyl is preferably a group having one, two, three or four double bonds, excluding 2-acyl-glycerol (20:4) (2-arachidonyl glycerol).

In this embodiment, the 2-glyceryl ethers preferably have a fatty acid moiety consisting of a 14 to 24 carbon atom backbone, such as 14, 15, 16, 17, 18, 19, 20, 21, 22, 23 or 24 carbon atoms, and between 0 and 4 double bonds, such as 0, 1, 2, 3 or 4 double bonds, excluding 2-arachydonyl glyceryl ether (2-glyceryl ether (20:4)). In other terms, "R" is C₁₄-C₂₄ alkyl or C₁₄-C₂₄ alkenyl, wherein alkenyl is preferably a group having one, two, three or four double bonds. Preferably, "R" is C₁₄-C₁₉ and C₂₁-C₂₄ alkyl or alkenyl, wherein alkenyl is preferably a group having one, two, three or four double bonds.

In this embodiment, the cannabinols preferably have a fatty alcohol moiety consisting of 1 to 4 and/or 6 to 24 carbon atoms, and preferably 1 to 4 carbon atoms, such as 1, 2, 3 or 4 carbon atoms. In other terms, "R" is C₁-C₄ and/or C₆-C2₄ alkyl or alkenyl, preferably C₁-C₄ such as C₁, C₂, C₃ or C₄ alkyl or alkenyl.

In this embodiment, the cannabidiols preferably have a fatty alcohol moiety consisting of a 1 to 4 and/or 6 to 24 carbon atom backbone, and preferably 1 to 4 carbon atoms, such as 1, 2, 3 or 4 carbon atoms. In other terms, "R" is C₁-C₄ and/or C₆-C2₄ alkyl or alkenyl, preferably C₁-C₄ such as C₁, C₂, C₃ or C₄ alkyl or alkenyl.

In this embodiment, the tetrahydrocannabinols preferably have a fatty alcohol moiety consisting of a 1 to 4 and/or 6 to 24 carbon atom backbone, and preferably 1 to 4 carbon atoms, such as 1, 2, 3 or 4 carbon atoms. In other terms, "R" is C₁-C₄ and/or C₆-C2₄ alkyl or alkenyl, preferably C₁-C₄ such as C₁, C₂, C₃ or C₄ alkyl or alkenyl.

The preferred embodiments of the endocannabinoids, cannabinoids and modified versions thereof described herein above for the first embodiment relating to endocannabinoids, cannabinoids and modified versions thereof which are not excluded in the present embodiment also apply *mutatis mutandis* to this embodiment.

In a preferred embodiment of the second embodiment, said one or more endocannabinoids, cannabinoids or modified versions thereof are complexed with a lipoprotein.

Lipoproteins and their complexing with endocannabinoids, cannabinoids and/or modified versions thereof have been described herein above.

In a preferred embodiment of the pharmaceutical compositions of the invention, the lipoprotein is a very low density lipoprotein (VLDL) and/or high density lipoprotein (HDL).

In another further preferred embodiment, the lipoprotein is a mixture of different lipoproteins such as the lipoproteins specifically described herein above. For example, a mixture of lipoproteins prepared using a KBr gradient from fetal calf serum can be employed. It is also envisaged to isolate single lipoprotein classes from mouse and/or human sources using KBr or Optiprep (Sigma-Aldrich) gradients.

In an additional preferred embodiment of the invention, and (a) in case the pharmaceutical compositions comprise one or more endocannabinoids selected from N-acylethanolamides, N-acyl dopamines and/or modified versions thereof, said pharmaceutical compositions further comprise one or more fatty acid amide hydrolase (FAAH) inhibitors; and/or (b) in case the pharmaceutical compositions comprise one or more 2-acyl glycerols and/or modified versions thereof said pharmaceutical compositions further comprise one or more monoacylglycerol lipase inhibitors.

In another preferred embodiment of the invention, the pharmaceutical compositions further comprise one or more hedgehog signaling pathway inhibitors.

The term "fatty acid amide hydrolase (FAAH) inhibitor" is known in the art to refer to inhibitors of the enzyme fatty acid amide hydrolase. Briefly, FAAH is a member of the serine hydrolase family of enzymes. It is known to be the principal catabolic enzyme for fatty acid amides, i.e. hydrolysing fatty acids. The term "inhibitor" refers to a compound having the capability to decrease the biological activity of the target, in this case the hydrolysing activity of FAAH, to at least 50%, preferably to at least 75%, more preferred to at least 90% and even more preferred to at least 95% such as at least 98% or even at least 99% or more. Said inhibitor may preferably be an antibody, an aptamer, an siRNA, an shRNA, an miRNA, a ribozyme, an antisense nucleic acid molecule, a peptide, polypeptide or a small molecule.
The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments as well as Fd, F(ab')₂, Fv or scFv fragments; see, for example Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also includes embodiments such as chimeric (human constant domain, non-human variable domain), single chain and humanized (human antibody with the exception of non-human CDRs) antibodies.

Various techniques for the production of antibodies are well known in the art and described, e.g. in Harlow and Lane (1988) and (1999), Ioc. cit. Thus, the antibodies can be produced by peptidomimetics. Further, techniques described for the production of single chain antibodies (see, inter alia, US Patent 4,946,778) can be adapted to produce single chain antibodies specific for the target of this invention. Also, transgenic animals or plants (see, e.g., US patent 6,080,560) may be used to express (humanized) antibodies specific for the target of this invention. Most preferably, the antibody is a monoclonal antibody, such as a human or humanized antibody. For the preparation of monoclonal antibodies, any technique which provides antibodies produced by continuous cell line cultures can be used. Examples for such techniques are described, e.g. in Harlow and Lane (1988) and (1999), Ioc. cit. and include the hybridoma technique (originally described by Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BlAcore system can be used to increase the efficiency of phage antibodies which bind to a target (Schier, Human Antibodies Hybridomas 7 (1996), 97-105; Malmborg, J. Immunol. Methods 183 (1995), 7-13). It is also envisaged in the context of this invention that the term "antibody" comprises antibody constructs which may be expressed in cells, e.g. antibody constructs which may be transfected and/or transduced via, inter alia, viruses or plasmid vectors.

Aptamers are nucleic acid molecules or peptide molecules that bind a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications (Osborne et. al. (1997), Current Opinion in Chemical Biology, 1:5-9; Stull & Szoka (1995), Pharmaceutical Research, 12, 4:465-483).

More specifically, aptamers can be classified as nucleic acid aptamers, such as DNA or RNA aptamers, or peptide aptamers. Whereas the former normally consist of (usually short) strands of oligonucleotides, the latter preferably consist of a short variable peptide domain, attached at both ends to a protein scaffold.

Nucleic acid aptamers are nucleic acid species that, as a rule, have been engineered through repeated rounds of in vitro selection or equivalently, SELEX (systematic evolution of ligands by exponential enrichment) to bind to various molecular targets such as small molecules, proteins, nucleic acids, and even cells, tissues and organisms.

Peptide aptamers usually are peptides or proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable peptide loop typically comprises 10 to 20 amino acids, and the scaffold may be any protein having good solubility properties. Currently, the bacterial protein Thioredoxin-A is the most commonly used scaffold protein, the variable peptide loop being inserted within the redox-active site, which is a -Cys-Gly-Pro-Cys-loop in the wild protein, the two cysteins lateral chains being able to form a disulfide bridge. Peptide aptamer selection can be made using different systems, but the most widely used is currently the yeast two-hybrid system.
Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are usually cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as in vivo diagnostic imaging. Several modifications, such as 2'-fluorine-substituted pyrimidines, polyethylene glycol (PEG) linkage, fusion to albumin or other half life extending proteins etc. are available to scientists such that the half-life of aptamers can be increased for several days or even weeks.

The term "peptide" as used herein describes a group of molecules consisting of up to 30 amino acids, whereas the term "polypeptide" as used herein describes a group of molecules consisting of more than 30 amino acids. The group of peptides and polypeptides are referred to together by the term "(poly)peptide". Also encompassed by the term "(poly)peptide" are proteins as well as fragments of proteins of more than 30 amino acids. The term "fragment of protein" in accordance with the present invention refers to a portion of a protein comprising at least the amino acid residues necessary to maintain the biological activity of the protein. Preferably, the amino acid chains are linear. (Poly)peptides may further form multimers consisting of at least two identical or different molecules. The corresponding higher order structures of such multimers are correspondingly termed homo- or heterodimers, homo- or heterotrimers etc. Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The term "(poly)peptide" also refers to naturally modified (poly)peptides where the modification is effected e.g. by glycosylation, acetylation, phosphorylation and similar modifications which are well known in the art.
It is also well known that (poly)peptides are not always entirely linear. For instance, (poly)peptides may be branched as a result of ubiquitination, and they may be circular, with or without branching, generally as a result of post-translation events, including natural processing event and events brought about by human manipulation which do not occur naturally. Circular, branched and branched circular (poly)peptides may be synthesized by non-translational natural processes and by synthetic methods. The modifications can be a function of how the (poly)peptide is made. For recombinant (poly)peptides, for example, the modifications will be determined by the host cells posttranslational modification capacity and the modification signals in the amino acid sequence. Accordingly, when glycosylation is desired, a (poly)peptide should be expressed in a glycosylating host, generally an eukaryotic cell, for example Cos7, HELA or others. The same type of modification may be present in the same or varying degree at several sites in a given (poly)peptide. Also, a given (poly)peptide may contain more than one type of modification.

In accordance with the present invention, the term "small interfering RNA (siRNA)", also known as short interfering RNA or silencing RNA, refers to a class of 18 to 30, preferably 19 to 25, most preferred 21 to 23 or even more preferably 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome.

siRNAs naturally found in nature have a well defined structure: a short double-strand of RNA (dsRNA) with 2-nt 3' overhangs on either end. Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Essentially any gene of which the sequence is known can thus be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. In general, any RNA molecule suitable to act as siRNA is envisioned in the present invention. The most efficient silencing was so far obtained with siRNA duplexes composed of 21-nt sense and 21-nt antisense strands, paired in a manner to have a 2-nt 3'-overhang. The sequence of the 2-nt 3' overhang makes a small contribution to the specificity of target recognition restricted to the unpaired nucleotide adjacent to the first base pair (Elbashir et al. 2001). 2'-deoxynucleotides in the 3' overhangs are as efficient as ribonucleotides, but are often cheaper to synthesize and probably more nuclease resistant. Delivery of siRNA may be accomplished using any of the methods known in the art, for example by combining the siRNA with saline and administering the combination intravenously or intranasally or by formulating siRNA in glucose (such as for example 5% glucose) or cationic lipids and polymers can be used for siRNA delivery in vivo through systemic routes either intravenously (IV) or intraperitoneally (IP) (Fougerolles et al. (2008), Current Opinion in Pharmacology, 8:280-285; Lu et al. (2008), Methods in Molecular Biology, vol. 437: Drug Delivery Systems - Chapter 3: Delivering Small Interfering RNA for Novel Therapeutics). The activity and specificity of siRNAs can be altered by various modifications such as, e.g., by inclusion of a blocking group at the 3' and 5' ends, wherein the term "blocking group refers to substituents of that can be attached to oligonucleotides or nucleomonomers, either as protecting groups or coupling groups for synthesis (cf. WO 98/13526, EP 2221377 B1), by inclusion of agents that enhance the affinity to the target sequence such as intercalating agents (e.g., acridine, chlorambucil, phenazinium, benzophenanthirdine), attaching a conjugating or complexing agent or encapsulating it to facilitate cellular uptake, or attaching targeting moieties for targeted delivery.

A short hairpin RNA (shRNA) is a sequence of RNA that makes a tight hairpin turn that can be used to silence gene expression via RNA interference. shRNA uses a vector introduced into cells and utilizes the U6 promoter to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs which match the siRNA that is bound to it. si/shRNAs to be used in the present invention are preferably chemically synthesized using appropriately protected ribonucleoside phosphoramidites and a conventional DNA/RNA synthesizer. Suppliers of RNA synthesis reagents are Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK). Most conveniently, siRNAs or shRNAs are obtained from commercial RNA oligo synthesis suppliers, which sell RNA-synthesis products of different quality and costs. In general, the RNAs applicable in the present invention are conventionally synthesized and are readily provided in a quality suitable for RNAi.

Further molecules effecting RNAi include, for example, microRNAs (miRNA). Said RNA species are single-stranded RNA molecules which, as endogenous RNA molecules, regulate gene expression. Binding to a complementary mRNA transcript triggers the degradation of said mRNA transcript through a process similar to RNA interference. Accordingly, miRNA may be employed, e.g., as an inhibitor of the Smo receptor.

A ribozyme (from ribonucleic acid enzyme, also called RNA enzyme or catalytic RNA) is an RNA molecule that catalyzes a chemical reaction. Many natural ribozymes catalyze either their own cleavage or the cleavage of other RNAs, but they have also been found to catalyze the aminotransferase activity of the ribosome. Non-limiting examples of well-characterized small self-cleaving RNAs are the hammerhead, hairpin, hepatitis delta virus, and in vitro-selected lead-dependent ribozymes, whereas the group I intron is an example for larger ribozymes. The principle of catalytic self-cleavage has become well established in the last 10 years. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each usually with 6 to 8 nucleotides, are taken and the catalytic hammerhead sequence is inserted between them. Molecules of this type were synthesized for numerous target sequences. They showed catalytic activity in vitro and in some cases also in vivo. The best results are usually obtained with short ribozymes and target sequences.

A recent development, also useful in accordance with the present invention, is the combination of an aptamer recognizing a small compound with a hammerhead ribozyme. The conformational change induced in the aptamer upon binding the target molecule is supposed to regulate the catalytic function of the ribozyme.

The term "antisense nucleic acid molecule" is known in the art and refers to a nucleic acid which is complementary to a target nucleic acid. An antisense molecule in accordance with the invention is capable of interacting with the target nucleic acid, more specifically it is capable of hybridizing with the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901).

A "small molecule" as used herein may be, for example, an organic molecule. Organic molecules relate or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively, the "small molecule" in accordance with the present invention may be an inorganic compound. Inorganic compounds are derived from mineral sources and include all compounds without carbon atoms (except carbon dioxide, carbon monoxide and carbonates). Preferably, the small molecule has a molecular weight of less than about 2000 amu, or less than about 1000 amu such as less than about 500 amu, and even more preferably less than about 250 amu. The size of a small molecule can be determined by methods well-known in the art, e.g., mass spectrometry. The small molecules may be designed, for example, based on the crystal structure of the target molecule, where sites presumably responsible for the biological activity, can be identified and verified in in vivo assays such as in vivo high-throughput screening (HTS) assays. Preferred small molecules are described herein and include SAG and cyclopamine as well as the further small molecules referred to herein below.

Exemplary FAAH inhibitors that can be used in accordance with the invention as well as methods to prepare corresponding inhibitors are described, e.g., in Ahn et al., Chemistry & Biology 16, 411-420 (2009) and references cited in the latter publication or Vandervoorde S., Curr Top Med Chem., 8(3):247-267 (2008).

The term "monoacylglycerol lipase" (also referred to in the art as MAG lipase or MAGL) is used according to its accepted meaning in the art, i.e. it refers to a membrane-associated member of the serine hydrolase superfamily and contains the classical GXSXG consensus sequence common to most serine hydrolases. MAGL functions *inter alia* together with hormone-sensitive lipase (LIPE) to hydrolyze intracellular triglyceride stores in adipocytes and other cells to fatty acids and glycerol. Also, it converts monoacylglycerols to the free fatty acid and glycerol. The term "inhibitor" has been defined herein above with regard to structure and function and said definition applies *mutatis mutandis.* Inhibitors of the enzyme monoacylglycerol lipase are, e.g., inhibitors such as *N-*arachidonoyl maleimide (NAM) 4-nitrophenyl 4-(dibenzo[*d*][1,3]dioxol-5-yl(hydroxyl)methyl)piperidine-1-carboxylate (JZL184) as described in Pan et al., JPET, 331:591-597 (2009), or inhibitors described by King et al., Chemistry & Biology, 16, 1045-1052 (2009); or inhibitors described by Ahn et al., Chemistry & Biology, 16, 411-420 (2009); or inhibitors described by Vandervoorde S., Curr Top Med Chem., 8(3):247-267 (2008).

Using one or more FAAH inhibitors and/or MAGL inhibitors as described herein above, is advantageous as such combinations decrease the rate of hydrolysis of the endocannabinoids specified in this embodiment and thereby increase the half life of the respective endocannabinoids and/or modified versions thereof that are part of a pharmaceutical composition according to the invention.

Various "hedgehog signaling pathway inhibitors" are known in the art, In particular those referred to herein above and are, e.g., described in Tremblay, M.R., et al., Current opinion in chemical biology, 2010. 14(3): p. 428-35; Lin, T.L. and W. Matsui, OncoTargets and therapy, 2012. 5: p. 47-58. Preferred Hh signaling pathway inhibitors are those that have been used as anti-tumour agents such as, e.g., GDC-0449 (Tradename: Erivedge, USAN generic name: vismodegib; PubChem identifier CID 24776445; available from Genentech, Curis and Roche), LDE-225 (USAN generic name: erismodegib; available from Novartis) and LEQ-506 (available from Novartis), BMS-833923 (available from Bristol-Myers-Squibb; also known as XL139 (available from Exelixis)), , IPI-926 (USAN generic name: saridegib; ChEMBL identifier: CHEMBL538867; available from Infinity), PF-0449913 (available from Pfizer), and TAK-441 (available from Milenium).

The pharmaceutical composition may comprise only one of the inhibitor species described herein or may comprise multiple inhibitor species from one or more inhibitor genera (antibody, aptamer, etc.). For example, at least (for each value) 2 such as 3, 4, 5, 6, 7 or 8, 9, 10 or more inhibitors from the same and/or different species and/or genus may be comprised in the pharmaceutical composition in accordance with the invention. It is understood that the inhibitor species may originate from one or more of the above described classes, i.e. FAAH inhibitors, monoacylglycerol lipase inhibitors, Hh signaling pathway inhibitors.

The function of any of the inhibitors referred to in accordance with the present invention may be identified and/or verified by using, e.g., high throughput screening assays (HTS). High-throughput assays, independently of being biochemical, cellular or other assays, generally may be performed in wells of microtiter plates, wherein each plate may contain, for example 96, 384 or 1536 wells, Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to the observed biological activity.

The determination of the binding of potential inhibitors can be effected in, for example and without limitation, any binding assay, preferably biophysical binding assay, which may be used to identify binding of test molecules prior to performing the functional/activity assay with the inhibitor. Suitable biophysical binding assays are known in the art and comprise fluorescence polarization (FP) assay, fluorescence resonance energy transfer (FRET) assay and surface plasmon resonance (SPR) assay. Instead of or in addition to assessing the direct interaction of inhibitor and target molecule by binding assays, one may indirectly determine the interaction of the inhibitor with its target molecule by using a suitable read out. For example, in cases where the inhibitor acts by decreasing the expression level of the target protein, the determination of the expression level of the protein can, for example, be carried out on the nucleic acid level or on the amino acid level.

Methods for determining the expression of a protein on the nucleic acid level include, but are not limited to, northern blotting, PCR, RT-PCR or real RT-PCR.
A northern blot allows the determination of RNA or isolated mRNA in a sample. Northern blotting involves the use of electrophoresis to separate RNA samples by size and detection with a hybridization probe complementary to part of or the entire target sequence. Initially, total RNA extraction from the sample is performed. If desired, mRNA can be separated from said initial RNA sample through the use of oligo (dT) cellulose chromatography to isolate only the RNA with a poly(A) tail. RNA samples are then separated by gel electrophoresis. The separated RNA is then transferred to a nylon membrane through a capillary or vacuum blotting system. After transfer to the membrane, the RNA is immobilized through covalent linkage to the membrane by, e.g., UV light or heat. Then, the RNA is detected using suitably labeled probes and X-ray film and can subsequently be quantified by densitometry. Suitable compositions of gels, buffers and labels are well-known in the art and may vary depending on the specific sample and target to be identified.
PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed, for example, in a 50 µl reaction mixture containing 5 µl of 10 x PCR buffer with 1.5 mM MgCl2, 200 µM of each deoxynucleoside triphosphate, 0.5 µl of each primer (10 µM), about 10 to 100ng of template DNA and 1 to 2.5 units of Taq polymerase. The primers for the amplification may be labeled or be unlabeled. DNA amplification can be performed, e.g., with a Applied Biosystems Veriti® Thermal Cycler (Life Technologies Corporation, Carlsbad, CA), C1000™ thermal cycler (Bio-Rad Laboratories, Hercules, CA,), or SureCycler 8800 (Agilent Technologies, Santa Clara, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step, e.g. at 55°C for 1 min as well as a final extension step, e.g. at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, it is well known in the art how to optimize PCR conditions for the amplification of specific nucleic acid molecules with primers of different length and/or composition or to scale down or increase the volume of the reaction mix. The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT reaction can be performed, for example, in a 20µl reaction mix containing: 4 µl of 5x AMV-RT buffer, 2 µl of oligo dT (100 µg/ml), 2µl of 10 mM dNTPs, 1µl total RNA, 10 Units of AMV reverse transcriptase, and H2O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case. The resulting products are loaded onto an agarose gel and band intensities are compared after staining the nucleic acid molecules with an intercalating dye such as ethidium bromide or SybrGreen. A lower band intensity of the sample treated with the inhibitor as compared to a non-treated sample indicates that the inhibitor successfully inhibits the protein.
Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor, which was formerly quenched due to the close proximity to the 3' acceptor in the TaqMan probe sequence. Thereby, the process of amplification can be monitored directly and in real time, which permits a significantly more precise determination of expression levels than conventional endpoint PCR. Also of use in real time RT-PCR experiments is a DNA intercalating dye such as SybrGreen for monitoring the de novo synthesis of double stranded DNA molecules.

Methods for the determination of the expression of a protein on the amino acid level include, but are not limited to, western blotting or polyacrylamide gel electrophoresis in conjunction with protein staining techniques such as Coomassie Brilliant blue or silver-staining. The total protein is loaded onto a polyacrylamide gel and electrophoresed. Afterwards, the separated proteins are transferred onto a membrane, e.g. a polyvinyldifluoride (PVDF) membrane, by applying an electrical current. The proteins on the membrane are exposed to an antibody specifically recognizing the protein of interest. After washing, a second antibody specifically recognizing the first antibody and carrying a readout system such as a fluorescent dye is applied. The amount of the protein of interest is determined by comparing the fluorescence intensity of the protein derived from the sample treated with the inhibitor and the protein derived from a non-treated sample. A lower fluorescence intensity of the protein derived from the sample treated with the inhibitor indicates a successful inhibitor of the protein. Also of use in protein quantification is the Agilent Bioanalyzer technique (e.g., Agilent 2100 Bioanalyzer; Agilent Technologies, Santa Clara, CA).

In a more preferred embodiment of the pharmaceutical compositions of the invention, the fatty acid amide hydrolase (FAAH) inhibitor is selected from the group consisting of PF-3845, URB597, URB754 and JZL195.

The FAAH inhibitor PF-3845 (N-3-Pyridinyl-4-[[3-[[5-(trifluoromethyl)-2-pyridinyl]oxy]phenyl]methyl]-1-piperidinecarboxamide hydrate; CAS number: 1196109-52-0) is an irreversible inhibitor that carbamylates FAAH's serine nucleophile. PF-3845 can be commercially obtained, e.g. through Sigma Aldrich. URB597 (also known as KDS-4103) is a FAAH inhibitor with the IUPAC name [3-(3-carbamoylphenyl)phenyl] N-cyclohexylcarbamate (ChEMBL number: CHEMBL184238) and commercially obtainable, e.g., through Sigma Aldrich. URB754 is regarded to be an FAAH inhibitor with the IUPAC name 6-methyl-2-[(4-methylphenyl)amino]-4H-3,1-benzoxazin-4-one (CAS number 86672-58-4). JZL195 has the IUPAC name of (4-nitrophenyl)4-[(3-phenoxyphenyl)methyl]piperazine-1-carboxylate.

In a preferred embodiment, the pharmaceutical composition further comprises a GPI-linked protein, a sterol-linked protein and/or a palmitoylated protein.

A "GPI-linked protein" is a protein to whose C-terminus a glycosylphophatidylinositol has been attached (also referred to as GPI anchor). It is composed of a phosphatidylinositol group linked through a carbohydrate-containing linker and via an ethanolamine phosphate (EtNP) bridge to the C-terminal amino acid of a mature protein. The two fatty acids within the hydrophobic phosphatidylinositol group enable anchoring such a protein into cell membranes and, as envisaged in accordance with the present invention, into lipoprotein membranes. Correspondingly anchored proteins may be released from the lipoproteins via, e.g., phospholipase C (PLC).

Sterols are a subgroup of steroids which due to their amphiphatic nature can integrate into cell membranes and, as envisaged in accordance with the present invention, into lipoprotein membranes. The process of linking a protein to a sterol is known in the art and, e.g., described in Brankatschk and Eaton, J. Neurosci., 30(31):10441-10447 (2010).

A palmitoylated protein is a protein that has been covalently attached to fatty acids, such as, e.g., palmitic acid, wherein said attachment can be effected, e.g., at cysteine, serine and/or threonine residues. The fatty acids enable anchoring such a protein into cell membranes and, as envisaged in accordance with the present invention, into lipoprotein membranes.

Various proteins may be modified and linked to sterols, GPI or fatty acids and used in the compositions of the invention. For example, proteins that allow targeting the lipoproteins complexed with endocannabinoids, cannabinoids and/or modified versions thereof comprised in the pharmaceutical composition to tumours can be used.

As evident from the above, this embodiment enables functionalisation of the lipoprotein(s) comprised in the pharmaceutical composition. For example, the bioavailability of the lipoproteins complexed with endocannabinoids, cannabinoids and/or modified versions thereof comprised in the pharmaceutical composition at the site of the tumour could be increased through using proteins that target said lipoproteins to said tumour. This way, one may also decrease any potential and unwanted systemic side effects.

In a more preferred embodiment, said one or more hedgehog signaling pathway inhibitors are selected from the group consisting of smoothened inhibitors and patched inhibitors.

As outlined herein above, smoothened and patched are two molecules at the top of the hedgehog signaling pathway that have shown to be valid targets for the inhibition of the hedgehog signaling pathway. A corresponding treatment setup is expected to be a valid strategy to avoid the development of resistances against hedgehog signaling pathway inhibitors.

In a further preferred embodiment of the pharmaceutical composition of the invention, the one or more endocannabinoids are selected from the group consisting of N-acylethanolamide (18:2), N-acylethanolamide (20:4), 2-glyceryl ether (16:0), 2-glyceryl ether (16:1), 2-glyceryl ether (18:1), 2-glyceryl ether (20:4), N-acyl dopamine (16:0), N-acyl dopamine (18:0) and/or modified versions thereof; and wherein the one or more cannabinoids are selected from the group consisting of cannabinol, cannabidiol and/or modified versions thereof.

The above named endocannabinoids and cannabinoids have been shown to be particularly potent antagonists of the hedgehog signaling pathway. The term "hedgehog signaling pathway" as used in accordance with the invention is well known in the art and comprehensively described, e.g., in Ryan and Chiang, J Biol Chem., 287(22):17905-17913 (2012); and in Bakarat et al., Trends Mol Med., 16(8):337-348 (2010).

In another embodiment, the invention relates to the use of the pharmaceutical composition of the main embodiment in the treatment of a tumour in a mammal.

Also, the invention relates to a method for treating a tumour in a mammal, wherein a pharmaceutical composition as defined herein above is administered to said mammal, thereby treating said tumour.

The term "mammal" is known in the art to refer to an animal that is grouped into the class of mammalia and used accordingly herein. Preferably, the mammal is a primate, more preferred a human.

The term "tumour" as used herein relates to neoplasms, e.g. characterized by abnormal tissue proliferation. Tumours that are known to metastasize are malignant tumours (also referred to in the art as cancerous tumours) in contrast to benign tumours that lack the ability to form metastases. However, many types of the latter tumours have the potential to become malignant, i.e. metastasize, (such as, e.g., teratoma). Tumours in accordance with the invention are i) carcinoma, ii) sarcoma, iii) carcinosarcoma, iv) tumours of the blood-forming tissues, v) tumours of the nerve tissue including the brain, and vi) cancer of skin cells. A tumour according to i) occurs in epithelial tissues, which cover the outer body (the skin) and line mucous membranes and the inner cavitary structures of organs e.g. such as the breast, lung, the respiratory and gastrointestinal tracts, the endocrine glands, and the genitourinary system. Ductal or glandular elements may persist in epithelial tumours, as in adenocarcinomas like, e.g., thyroid adenocarcinoma, gastric adenocarcinoma, uterine adenocarcinoma. Tumours of the pavement-cell epithelium of the skin and of certain mucous membranes, such as e. g. tumours of the tongue, lip, larynx, urinary bladder, uterine cervix, or penis, may be termed epidermoid or squamous-cell carcinomas of the respective tissues and are in the scope of the definition of tumour as well. A tumour according to ii) develops in connective tissues, including fibrous tissues, adipose (fat) tissues, muscle, blood vessels, bone, and cartilage like e.g. osteogenic sarcoma, liposarcoma, fibrosarcoma, or synovial sarcoma. A tumour according to iii) is a tumour that develops in both epithelial and connective tissue. A tumour within the scope of this definition may be a primary or secondary tumour, whereby "primary" indicates that the tumour originated in the tissue where it is found rather than was established as a secondary site through metastasis from another tumour lesion. As outlined above, tumours within the scope of this definition may be benign or malign and may affect all anatomical structures of the body of a subject, preferably a mammal. For example, they comprise tumours of a) the bone marrow and bone marrow derived cells (leukemias), b) the endocrine and exocrine glands like e. g. thyroid, parathyroid, pituitary, adrenal glands, salivary glands, pancreas, c) the breast, like e. g. benign or malignant tumours in the mammary glands of either a male or a female, the mammary ducts, adenocarcinoma, medullary carcinoma, comedo carcinoma, Paget's disease of the nipple, inflammatory carcinoma of the young woman, d) the lung, e) the stomach, f) the liver and spleen, g) the small intestine, h) the colon, i) the bone and its supportive and connective tissues like malignant or benign bone tumour, e. g. malignant osteogenic sarcoma, benign osteoma, cartilage tumors; like malignant chondrosarcoma or benign chondroma; bone marrow tumours like malignant myeloma or benign eosinophilic granuloma, as well as metastatic tumors from bone tissues at other locations of the body; k) the mouth, throat, larynx, and the esophagus, l) the urinary bladder and the internal and external organs and structures of the urogenital system of male and female like ovaries, uterus, cervix of the uterus, testes, and prostate gland, m) the prostate, n) the pancreas, like ductal carcinoma of the pancreas; o) the lymphatic tissue like lymphomas and other tumors of lymphoid origin, p) the skin, q) cancers and tumor diseases of all anatomical structures belonging to the respiration and respiratory systems including thoracal muscles and linings, r) primary or secondary cancer of the lymph nodes s) the tongue and of the bony structures of the hard palate or sinuses, t) the mouth, cheeks, neck and salivary glands, u) the blood vessels including the heart and their linings, v) the smooth or skeletal muscles and their ligaments and linings, w) the peripheral, the autonomous, the central nervous system including the cerebellum, x) the adipose tissue. The tumours may be in any stage of their development such as early stages, where the tumour is still confined to the tissue of origin (or primary site), or when the cancer cells have already invaded adjacent tissue or distant parts of the body from the primary site, i.e. the tumour already metastasizes. Metastasis of tumors is the process in which cancer cells can detach from the primary tumor and produce daughter tumors either locally or after spreading via blood or lymphatic vessels. Tumors which have a high incidence of metastasis are e.g. tumors of the lung, breast, colon, kidney, prostate, pancreas, cervix, as well as melanoma.

It has been shown prior to the invention that endocannabinoids and cannabinoids show anti-tumour effects in various tumour cells, such as, e.g. neuroblastoma, mantle cell lymphoma, colon cancer, osteosarcoma, glioma (e.g. Hamtiaux et al., BMC Cancer, 12:92 (2012); Flygare et al., FEBS Lett., 579(30):6885-6889 (2005); Bifulco et al., FASEB J., 18(13):1606-1608 (2004); Bifulco and Di Marzo, Nature Medicine, vol. 8, no. 6, (2002)). Therefore, the treatment of tumours according to the invention is a viable option for the medical practitioner, in particular with regard to the finding that endocannabinoids, cannabinoids and/or modified versions thereof as defined herein are hedgehog antagonists and can be employed by taking advantage of their endogenous delivery mechanism, i.e. in complex with lipoproteins.

In a preferred embodiment, the tumour is a hedgehog signaling-dependent tumour.

The term "hedgehog signaling-dependent tumour" relates to tumours (i) which develop due to abnormal (disregulated) hedgehog signaling and can be treated by hedgehog signaling antagonists, and/or (ii) whose viability such as, e.g., proliferation, survival, progression, capability to metastasize can be diminished by targeting the hedgehog signaling pathway. In other words, the term relates to tumours whose initiation and/or viability relies at least to some extent on the hedgehog signaling pathway. Various scenarios exist that characterise on a molecular level a corresponding dependence of tumours on the hedgehog signaling pathway which have been described, e.g., in and in Bakarat et al., Trends Mol Med., 16(8):337-348 (2010); in Raju and Pham, Vitam Horm., 88:507-522 (2012). The above described disregulations of the hedgehog signaling pathway can, e.g., occur within the tumour cells themselves or in the surrounding tissue thereby providing a suitable environment for the viability of said tumour. Accordingly, Hedgehog signaling-dependent tumours are tumours where the Hedgehog signaling pathway has been activated upstream from or at the level of Smoothened (Smo). This includes tumours that express the hedgehog ligands Shh (Sonic hedgehog), Dhh (Desert hedgehog) or Ihh (Indian hedgehog) themselves, tumours that have lost Patched (Ptc) function and/or tumours with activating mutations in Smoothened (Smo) (see e.g., Yauch et al., Nature, 455, 406-411 (2008)). Specific tumours are mentioned in the following: about 90% of Basal Cell Carcinomas (BCC) have lost Ptc and most of the rest have activating mutations in Smoothened. Phase I clinical trials of Smoothened inhibitors have shown dramatic results against metastatic BCC (Ng and Curran, Nature Reviews, Cancer, 11 (7): p. 493-501 (2011)).
30% of Medulloblastomas show evidence for Hh pathway activation and about half of these appear to have lost Ptc. Phase 1 clinical trials of Smoothened inhibitors have already shown dramatic activity towards medulloblastomas (Ng and Curran, Nature Reviews, Cancer, 11 (7): p. 493-501 (2011)).
Many tumours of the gastrointestinal tract (derived from esophagus, stomach pancreas and bile duct) express Hh ligands. Cancer lines from these tumours are growth inhibited by cyclopamine and, e.g., a bile duct tumor line responds to cyclopamine treatment in a xenograft model (Berman et al., Nature, 425(6960): p. 846-51 (2003). Subsequent work on pancreatic cancer has shown two mechanisms that can be affected by Hh pathway inhibition: Smo antagonists kill pancreatic cancer stem cells (Singh et al., PloS one, 6(11): p. e27306 (2011)) in vitro, and can also act on the stroma surrounding the tumor in vivo to interfere with its support of the tumor and allow better access of other chemotherapeutic agents (Olive et al., Science, 324(5933): p. 1457-61 (2009)).
Ovarian cancer lines express Hh ligands and Smo antagonists reduce tumor growth in xenograft models, acting both in tumor and stroma (McCann et al., PloS one, 6(11): p. e28077 (2011); Bhattacharya et al., Clinical Cancer Research : an official journal of the American Association for Cancer Research, 14(23): p. 7659-66 (2008)).
Many prostate cancers express Sonic Hedgehog, and inactivating anti-Shh antibodies. Also, cyclopamine inhibits their proliferation in vitro.
A subset of small cell lung carcinoma lines express Hh ligands, and the proliferation of these lines is sensitive to cyclopamine (Yuan et al., Oncogene, 26(7): p. 1046-55 (2007)).
Breast cancer cell lines express both Shh and Hh pathway components and their proliferation is sensitive to cyclopamine (Kubo et al., Cancer research, 64(17): p. 6071-4 (2004)).
Loss of Smo contributes to growth of chronic myeloid leukemia (CML) stem cells. CML growth is inhibited in xenograft models by Smoothened antagonists (Zhao et al., Nature, 458(7239): p. 776-9 (2009)).
Multiple Myeloma consists of two populations - the differentiated plasma cells, and a population with stem cell markers. The latter expresses Hh ligands and Hh pathway components, and shows evidence of pathway activation. Their growth and clonogenic potential is inhibited in vitro by inactivating antibodies to Hh ligands, and by Smo antagonists (Peacock et al., PNAS, 104(10): p. 4048-53 (2007)).
Many hepatocellular carcinoma lines express Hh ligands and pathway components. In these cases, addition of Hh inactivating antibodies or Smo antagonists inhibits proliferation and induces apoptosis (Huang, et al., Carcinogenesis, 27(7): p. 1334-40 (2006)).
The Hh pathway is activated in progenitor cells of two different subtypes of glioma, and their proliferation in culture depends on addition of Hh Ligands. Normally, Hh ligands are provided by tissue adjacent to the tumour in vivo (Ehtesham et al., Oncogene, 26(39): p. 5752-61 (2007)). Treatment with Smo antagonists confers a survival advantage in xenograft models of Hh-dependent glioma (Sarangi et al., Oncogene, 28(39): p. 3468-76 (2009)).
All the above described types of tumours are considered hedgehog signalling-dependent tumours which can be treated in accordance with the invention.
Determination as to whether a tumour is likely to be hedgehog signaling-dependent can be performed by known methods such as qPCR or immunohistochemistry of Hh ligands and pathway components to determine expression, and sequencing of genes in the Hh pathway to identify mutations deletions or amplifications. This is described, e.g., in Berman et al., Nature, 425, 846-851 (2003); Yasui et al., Gastric Cancer, 4:113-121 (2001); Lau et al., Childs Nerv Syst, 28:521-532 (2012)).

As is evident from the above, the involvement of the hedgehog signaling pathway in the genesis and persistence of tumours is well-established in the art, as is its use as a pharmaceutical target to treat hedgehog signaling-dependent tumours. Accordingly, the treatment of tumours according to the invention by equally targeting the hedgehog signaling pathway is a viable option for the medical practitioner.

In a further embodiment, the invention relates to the use of the pharmaceutical composition in the treatment of a tumour in a mammal, wherein said tumour is a hedgehog signaling-dependent tumour. All definitions given herein, in particular above, also apply *mutatis mutandis* to this embodiment.

Also, the invention relates to a method for treating a hedgehog signaling-dependent tumour in a mammal, wherein a pharmaceutical composition as defined herein above is administered to said mammal, thereby treating said hedgehog signaling-dependent tumour. All definitions given herein, in particular above, also apply *mutatis mutandis* to this embodiment.

In a preferred embodiment, the hedgehog signaling-dependent tumour is selected from the group consisting of ovarian cancer, transitional cell carcinoma of the bladder, basal cell carcinoma, medulloblastoma, chronic myeloid leukemia, esophageal cancer and multiple myeloma.

In a further embodiment, the invention relates to a method for modulating the activity of the hedgehog signaling pathway in a mammalian cell, the method comprising the step of: contacting said cell with one or more endocannabinoids selected from (a) the group consisting of N-acylethanolamides, n-acyl-dopamines, 2-acyl-glycerols, 2-glyceryl-ethers and/or modified versions thereof; or (b) the group consisting of N-acyl serines and/or modified versions thereof; and/or contacting said cell with one or more cannabinoids selected from cannabinols, cannabidiols, tetrahydrocannabinols and/or modified versions thereof, thereby modulating the activity of the hedgehog signaling pathway in said mammalian cell.

The definitions given herein above also apply to this embodiment *mutatis mutandis.*

The endocannabinoid class of "N-acyl serines" is also well known in the art (Milman et al., PNAS, 103, 7, 2428-2433 (2006); Zhang et al., British Journal of Pharmacology, 160, 1583-1594 (2010)). Briefly, an N-acyl serine is made up of serine that is amide linked to a fatty acid moiety. The structural formula is depicted below, wherein "R" is selected from C₁-C₃₀ alkyl, C₂-C₃₀ alkenyl or C₂-C₃₀ alkynyl, optionally substituted with substituents. Preferably, "R" is C₁-C₃₀ alkyl or C₂-C₃₀ alkenyl. Also preferred, "R" is C₁₆-C₁₈ alkyl or C₁₆-C₁₈ alkenyl, such as C₁₆, C₁₇ or C₁₈ alkyl or alkenyl. Alkenyl is preferably a group having one, two, three or four double bonds.Preferably, the N-acyl serines are N-acyl serine (16:0) and (18:1).
N-acyl serines can be obtained commercially, e.g., from Avanti Polar Lipids, Tocris Bioscience, Biomol, Cayman Chemicals, Sigma-Aldrich or Fluka.

The generic structural formula for N-acyl serines is as follows:

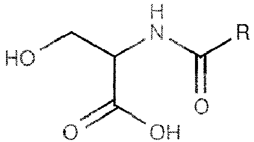

In accordance with the present invention, the term "modulating" relates to the effect conferred by the endocannabinoids, cannabinoids and/or modified versions thereof to alter, i.e. increase or decrease, the biological function or activity of the hedgehog signaling pathway. Said biological function or activity of the hedgehog signaling pathway may be, e.g., to promote growth or survival, or to direct differentiation down particular paths. Hh signaling specifies the identity of neurons, positional identity of cells in the limb bud.

The cell in this embodiment can be any mammalian cell comprising a hedgehog signaling pathway. Preferably, the cell endogenously comprises a hedgehog signaling pathway, i.e. minimally the pathway molecules Patched, Smoothened, Fused, Suppressor of Fused, Costal2, Gli1, Gli2, Gli3but also recombinant expression of the hedgehog pathway molecules is envisaged. It is understood that depending on the goal to be achieved with the method described, different cells may be more suitable than others. Preferably, the mammalian cell is a human cell which is not a human embryonic stem cell. The term "mammalian cell" as used herein, is well known in the art and refers to any cell belonging to an animal that is grouped into the class of mammalia. The term "cell" as used herein can refer to a single and/or isolated cell or to a cell that is part of a multicellular entity such as a tissue, an organism or a cell culture. In other words the method can be performed *in vivo,* ex *vivo* or *in vitro.* The method preferably excludes methods of treatment of the human or animal body or diagnostic methods practiced on the human or animal body. Depending on the particular goal to be achieved with the method of the invention, cells of different mammalian subclasses such as prototheria or theria may be used. For example, within the subclass of theria, preferably cells of animals of the infraclass eutheria, more preferably of the order primates, artiodactyla, perissodactyla, rodentia and lagomorpha are used in the method of the invention. Furthermore, within a species one may choose a cell to be used in the method of the invention based on the tissue type and/or capacity to differentiate equally depending on the goal to be achieved by altering the genome. Three basic categories of cells make up the mammalian body: germ cells, somatic cells and stem cells. A germ cell is a cell that gives rise to gametes and thus is continuous through the generations. Stem cells can divide and differentiate into diverse specialized cell types as well as self renew to produce more stem cells. In mammals there are two main types of stem cells: embryonic stem cells and adult stem cells. Somatic cells include all cells that are not a gametes, gametocytes or undifferentiated stem cells. The cells of a mammal can also be grouped by their ability to differentiate. A totipotent (also known as omnipotent) cell is a cell that is able to differentiate into all cell types of an adult organism including placental tissue such as a zygote (fertilized oocyte) and subsequent blastomeres, whereas pluripotent cells, such as embryonic stem cells, cannot contribute to extraembryonic tissue such as the placenta, but have the potential to differentiate into any of the three germ layers endoderm, mesoderm and ectoderm. Multipotent progenitor cells have the potential to give rise to cells from multiple, but limited number of cell lineages. Further, there are oligopotent cells that can develop into only a few cell types and unipotent cells (also sometimes termed a precursor cell) that can develop into only one cell type. There are four basic types of tissues: muscle tissue, nervous tissue, connective tissue and epithelial tissue that a cell to be used in the method of the invention can be derived from, such as for example hematopoietic stem cells or neuronal stem cells. To the extent human cells are envisaged for use in the method of the invention, it is preferred that such human cell is not obtained from a human embryo, in particular not via methods entailing destruction of a human embryo (corresponding methods are available as of 2008). On the other hand, human embryonic stem cells are at the skilled person's disposal such as taken from existent embryonic stem cell lines commercially available. Accordingly, the present invention may be worked with human embryonic stem cells without any need to use or destroy a human embryo. Alternatively, or instead of human embryonic stem cells, pluripotent cells that resemble embryonic stem cells such induced pluripotent stem (iPS) cells may be used, the generation of which is state of the art (Hargus G et al., Proc Natl Acad Sci U S A 107:15921-15926; Jaenisch R and Young R., 2008, Cell 132:567-582; Saha K, and Jaenisch R., 2009, Cell Stem Cell 5:584-595.

Cells to be used may originate from established cell lines but may also include cell of a primary cell line established from a tissue sample. Preferably, the cells originate from the same cell line or are established from the same tissue. Methods to obtain samples from various tissues and methods to establish primary cell lines are well-known in the art (Jones GE, Wise CJ., "Establishment, maintenance, and cloning of human dermal fibroblasts." Methods Mol Biol. 1997;75:13-21). Suitable cell lines may also be purchased from a number of suppliers such as, for example, the American tissue culture collection (ATCC), the German Collection of Microorganisms and Cell Cultures (DSMZ) or PromoCell GmbH, Sickingenstr. 63/65, D-69126 Heidelberg.

The term "contacting" is meant to refer to the process of bringing the endocannabinoids, cannabinoids and modified versions thereof in physical proximity with the cell so that they can modulate the hedgehog signaling pathway. Thus, contacting the cell encompasses, e.g., the mixing with or the applying of the endocannabinoids, cannabinoids and modified versions thereof.

In a preferred embodiment of the above method of the invention, the endocannabinoids, cannabinoids and/or modified versions thereof are brought into contact with the cells after having been complexed with lipoproteins as described herein above.

The findings presented herein establish the endocannabinoids and cannabinoids referred to in this embodiment as capable of modulating the hedgehog signaling pathway. As such, the method described herein will be applicable in a variety of scenarios such as, e.g., validating pharmaceutical targets or drugs.

In a further embodiment, the invention relates to the use of one or more endocannabinoids selected from (a) the group consisting of N-acylethanolamides, N-acyl dopamines, 2-acyl-glycerols, 2-glyceryl-ethers and/or modified versions thereof; (b) the group consisting of N-acyl serines and/or modified versions thereof; and/or cannabinoids selected from the group consisting of cannabinols, cannabidiols, tetrahydrocannabinols and/or modified versions thereof in the differentiation of embryonic stem cells, progenitor cells and/or induced stem cells.

Embryonic stem cells, progenitor cell and induced stem cells have been described herein above.
The term "differentiation" in the context of embryonic stem cells, progenitor cells and induced stem cells refers to the development of said cells towards a more determined cell fate. For example, embryonic stem cells can mimic normal embryonic development and differentiate into any kind of cell. The Hedgehog signaling pathway is used in many different contexts during normal development to specify the position of cells in a developmental field, allowing them to activate appropriate patterns of differentiation. Hedgehog acts as a morphogen, i.e., it is produced in a restricted region of the tissue, and spreads to form a concentration gradient such that cells at different distances from the source are exposed to different levels of Hedgehog signaling. The specific differentiation pathway followed by the cells depends on the concentration of Hedgehog morphogen, and on their previous history. For example, Hh signaling in the developing neural tube specifies different types of neurons. It is known that both Hh ligands and Smo antagonists have been used to differentiate IPS and ES cells to desired cell types, so endocannabinoids, cannabinoids and/or modified versions thereof can be used for the same purpose as envisage in this embodiment.
Pancreatic insulin producing cells have been derived from ES cells by a protocol that includes addition of cyclopamine (Jaramillo and Banerjee, Journal of visualized Experiments: JoVE, 61 (2012)). Therefore, the endocannabinoids of (a), cannabinoids and/or modified versions thereof which act as hedgehog signaling antagonists, e.g., endocannabinoids like 2-gylceryl ether (20:4), N-acylethanolamide (18:2) or (20:4), or N-acyl dopamine (18:0) or (16:0) are equally expected to be effective in the derivation of pancreatic insulin producing cells from ES cells.
Shh has been used to promote differentiation of IPS cells to retinal pigment epithelial cells (Zahabi et al., Stem cells and Development, 2012). Shh itself, or Smo agonists in general have been used to promote neuronal differentiation of human IPS cells (Mak et al., Stem Cells International, 2012: p. 140427 (2012)). Dopaminergic neurons have been differentiated from ES and IPS cells by a protocol that includes addition of Shh (Lau et al., Neuroreport, 17(10): p. 975-9 (2006); Xi et al., Stem cells, 30(8):1655-1663 (2012)). Motor neurons have been produced from ES and IPS cells by protocols that include addition of Shh and Smo agonist, (Wu et al., Journal of visualized Experiments : JoVE, 64 (2012); Lim et al., Current Neurovascular Research, 3(4): p. 281-8 (2006); Hu and Zhang, Nature Protocols, 4(9): p. 1295-304 (2009)). Also, intrathecal injection of Shh has been shown to improve recovery after induction of ischemic stroke in the brains of mouse (Bambakidis et al., Journal of Neurosurgery, 116(5): p. 1114-20 (2012)). Therefore, the use of N-acyl serines, which stimulate the Hh pathway, is equally considered to be effective the above mentioned purposes.
The same limitations regarding the use of human cells as described herein above apply *mutatis mutandis* to this embodiment.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all attached claims.

The figures show:

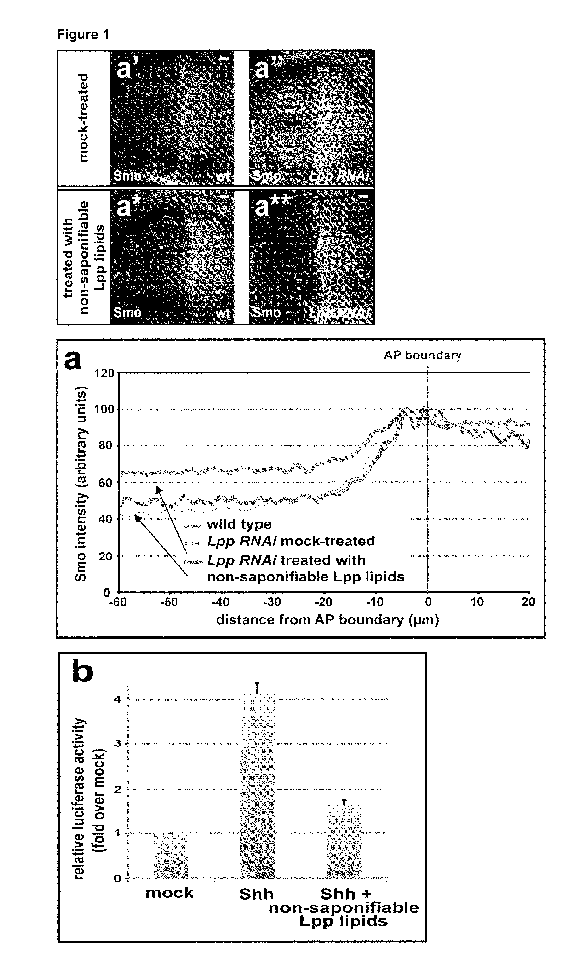

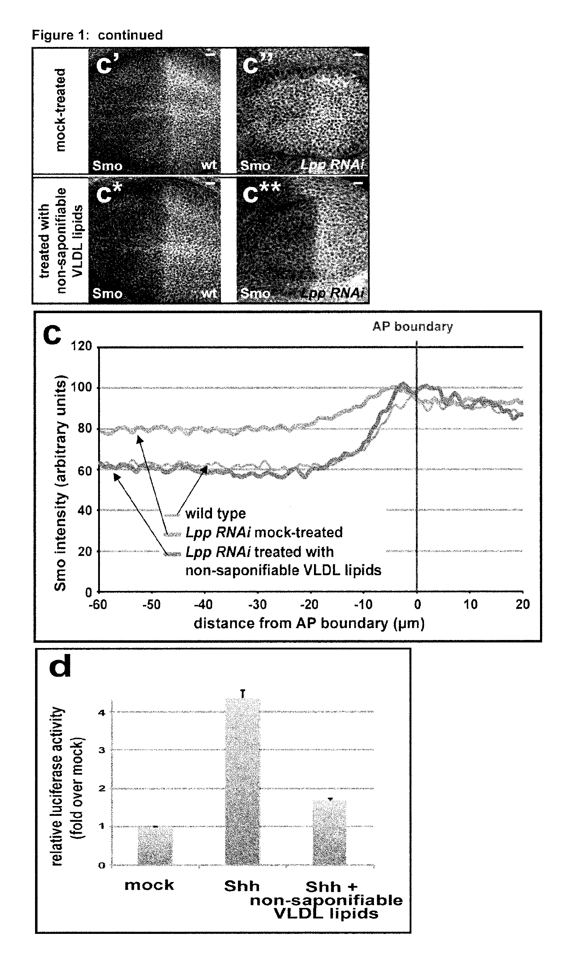

**Figure 1****: (a,b) Non-saponifiable Lpp lipids are needed for Ptc-mediated Smo destabilization in the wing disc**
   (a'-a**) Wing discs dissected from either wild type (a',a*) or LppRNAi (a",a**) animals were incubated with liposomes consisting of DOPC (mock) or DOPC + non-saponifiable Lpp lipids, at concentrations corresponding to those present in the hemolymph. After 2 hours at room temperature, discs were fixed and stained for Smo. In wild type mock-treated discs (a'), Smo is destabilized post-translationally by Patched in the anterior compartment. In mock-treated discs from Lpp knock-down animals (a") Ptc-mediated Smo destabilization is inhibited. Incubating LppRNAi discs with non-saponifiable Lpp lipids (a**) reverses ectopic Smo accumulation in the anterior compartment, but does not affect Smo levels in wild type discs (a*). Scale bar = 10µM.
   (a) Quantification of Smo staining in mock-treated discs from wild type (grey) and LppRNAi (orange) animals, and LppRNAi discs incubated with non-saponifiable Lpp lipids (blue). n>10 discs for each quantification.
   (b) Non-saponifiable Drosophila Lpp lipids inhibit signaling in ShhLIGHT2 cells.
      ShhLIGHT2 cells express Firefly luciferase under the control of a Gli responsive promoter, and Renilla Luciferase controlled by the viral Thymidine kinase promoter. The ratio of Firefly to Renilla Luciferase activity is a specific measure of Hh pathway activity. This panel shows the ratio of Firefly:Renilla Luciferase activity in ShhLIGHT2 cells treated in serum-free medium with (1) untransfected serum-free HeLa cell supernatants (mock), (2) with non-lipoprotein-associated Shh obtained from Shh-transfected HeLa cells grown in serum-free media. HeLa derived Shh + non-saponifiable Lpp lipids. These lipids were added in DMSO at a concentration that mimicked that present in the larval hemolymph. Error bars indicate standard deviation of 3 independent experiments.
   (c,d) Non-saponifiable VLDL lipids reverse ectopic Smo stabilization in LppRNAi wing discs.
   (c'-c**) Wing discs dissected from either wild type (c',c*) or LppRNAi (c",c**) animals were incubated with liposomes consisting of DOPC (mock) or DOPC + non-saponifiable VLDL lipids, at concentrations corresponding to those present in human plasma. After 2 hours at room temperature, discs were fixed and stained for Smo. Incubating LppRNAi discs with non-saponifiable VLDL lipids (c**) reverses ectopic Smo accumulation in the anterior compartment caused by LppRNAi (c"). Scale bar = 10µM.
   (c) Quantification of Smo staining in mock-treated discs from wild type (grey) and LppRNAi (orange) animals, and LppRNAi discs incubated with non-saponifiable VLDL lipids (green). n>10 discs for each quantification.
   (d) Non-saponifiable VLDL lipids inhibit signaling in ShhLIGHT2 cells.
   This panel shows the normalized ratio of Firefly:Renilla Luciferase activity in ShhLIGHT2 cells treated in serum-free medium with untransfected serum-free HeLa cell supernatants (mock) or with non-lipoprotein-associated Shh obtained from Shh-transfected HeLa cells grown in serum-free media. HeLa derived Shh + non-saponifiable VLDL lipids. The Firefly:Renilla ratio is normalized to that of mock-treated cells. These lipids were added in DMSO at a concentration that mimicked that present in human plasma. Error bars indicate standard deviation of 3 independent experiments.

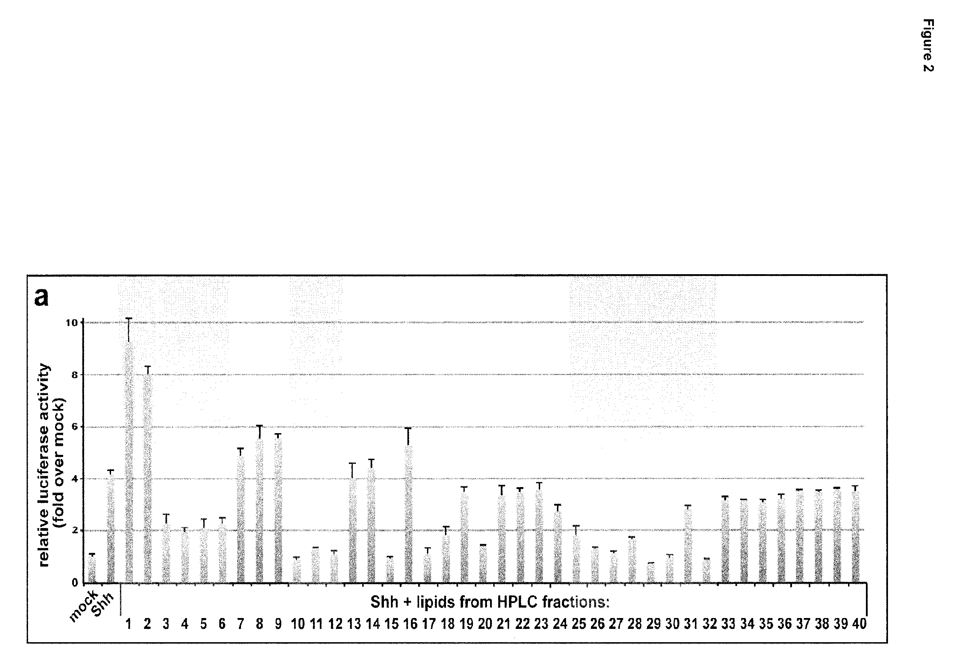

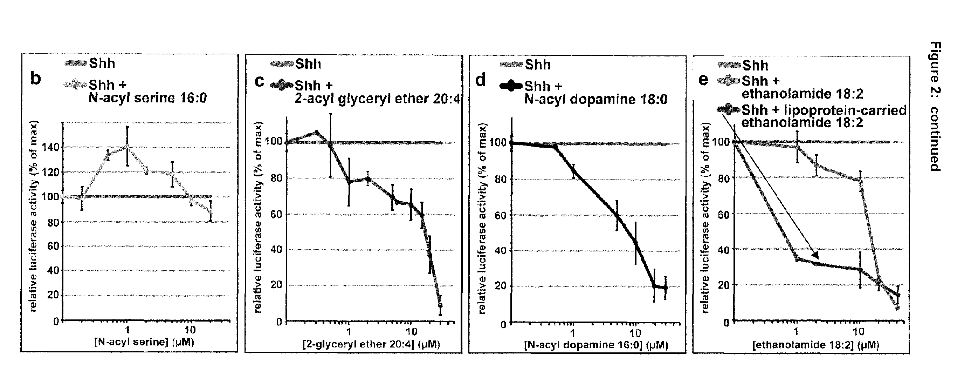

**Figure 2****: Specific endocannabinoid species in VLDL inhibit signaling in ShhLIGHT2 cells**
   (a) Lipids extracted from VLDL were saponified, loaded onto a C18 column in 60% methanol in H2O and eluted with a step gradient from 60% to 80% methanol in H2O, followed by an isocratic step of 80% methanol in H2O and a linear gradient of 80% to 100% methanol in H2O. Fractions were added to ShhLIGHT2 cells treated with non-lipoprotein-associated Shh made in HeLa cells, and resulting Hh pathway activity was measured as the Firefly:Renilla ratio normalized to the ratio in cells that were not stimulated with Shh. Error bars indicate standard deviations of three separate measurements of fraction activity.
   (b-d) Effects of N-acyl serine (16:0) (b), N-acyl dopamine (18:0) (c) and 2-glyceryl ether (20:4) (d) on Shh signaling in ShhLIGHT2 cells. For each experiment, the Firefly:Renilla ratio in cells not treated with Shh is defined as 0 to 0, and the fold increase in the Firefly:Renilla ratio obtained by addition of Shh is normalized to 100. Fold stimulation by Shh in these experiments ranged from 3-5. Error bars indicate standard deviations of at least 5 independent experiments.
      N-acyl serine (16:0) increases Shh signaling by 40% with an EC₅₀ of 0.5 µM. 2-acyl glyceryl ether (20:4) inhibits Shh signaling almost completely at 30µM, with an EC₅₀ of 15µM. N-acyl dopamine (18:0) inhibits Shh signaling by 80% at 30 µM, with an EC₅₀ of 5 µM. Ethanolamide (18:2) inhibits Shh signaling almost completely at 30µM, with an EC₅₀ of approximately 15 µM.
   (e) Effect of increasing concentrations of ethanolamide (18:2) on signaling by Shh in ShhLIGHT2 cells added in DMSO without prior incubation (red) or after pre-incubation with lipoproteins (see arrow). The EC₅₀ of ethanolamide (18:2) is decreased over 10-fold by pre-incubation with lipoproteins. Error bars indicate standard deviations of 3 independent experiments.

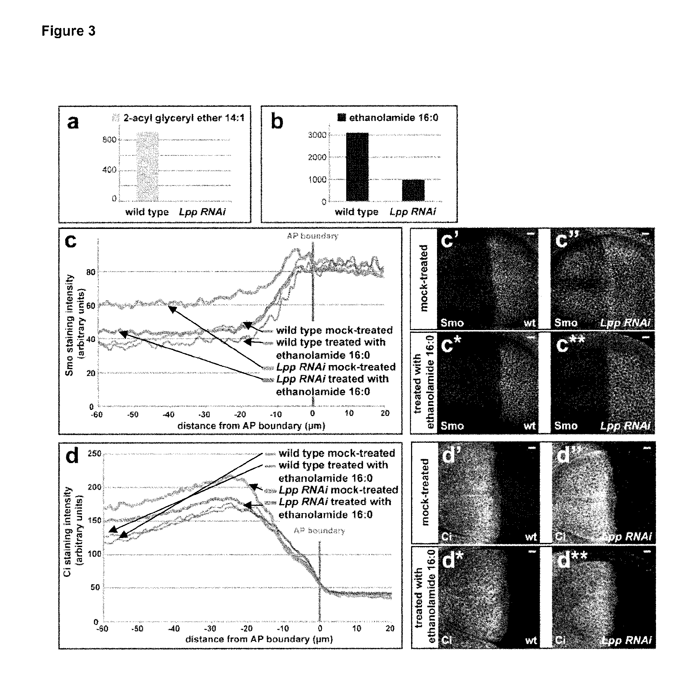

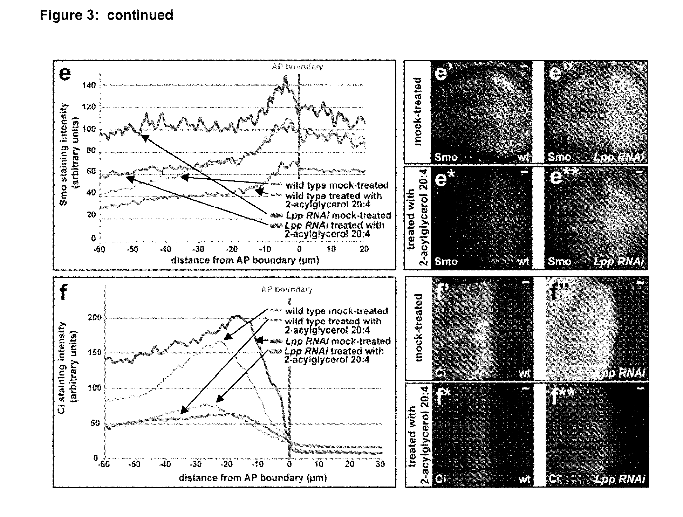

**Figure 3****: Endocannabinoids are present in Lipophorin and destabilize Smo in imaginal discs**
   (a,b) Shows relative levels of 2-glyceryl ether (14:1) (a) and N-acylethanolamide (16:0) (b) detected by MS/MS in hemolymph of wild type and LppRNAi animals.
   (c-d**) show immunofluorescence images (c'-c**,d'-d**) and corresponding staining quantifications (c,d) of wing imaginal discs from wild type (c',c*,d',d*) and LppRNAi (c",c**,d",d**) animals that have been incubated with DOPC liposomes (mock, c',c",d',d") or DOPC liposomes containing N-acylethanolamide (16:0) (c*,c**,d*,d**). After two hours, incubated discs were stained for Smo (c'-c**) or Ci155 (d'-d**). N-acylethanolamide (16:0) reverses ectopic Smo stabilization caused by LppRNAi and partially reverses Ci155 accumulation. Scale bar = 10µM. n>10 discs for each quantification. (e-f**) show immunofluorescence images (e'-e**,f'-f**) and corresponding staining quantifications (e,f) of wing imaginal discs from wild type (e',e*,f',f*) and LppRNAi (e",e**,f',f**) animals that have been incubated with DOPC liposomes (mock, e',e",f',f') or DOPC liposomes containing 2-acyl glycerol (20:4) (e*,e**,f*,f**). After two hours, incubated discs were stained for Smo (e'-e**) or Ci155 (f'-f**). 2-acyl glycerol (20:4) completely reverses ectopic Smo stabilization caused by LppRNAi and dramatically reduces Ci₁₅₅ accumulation in both LppRNAi (f**) and wild type (f*) discs to levels less than wild type (f and compare f with f*,f**). Scale bar = 10µM. n>10 discs for each quantification.

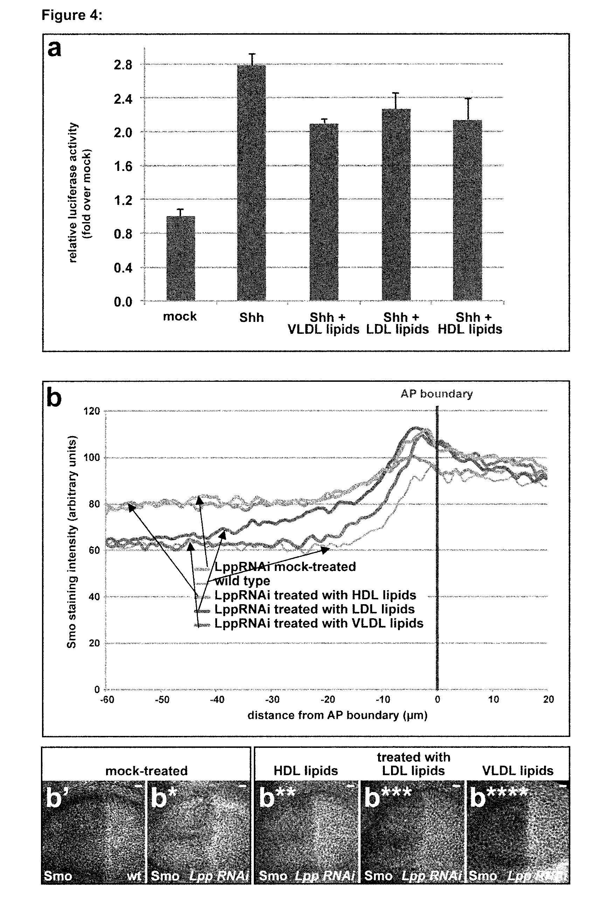

**Figure 4****: Lipids derived from VLDL and Lipophorin regulate Smo stability and Shh signaling in LIGHT2 cells**
   (a) Lipids derived from HDL, LDL and VLDL inhibit signaling by ShhN* in ShhLIGHT2 cells. ShhLIGHT2 cells express Firefly luciferase under the control of a Gli responsive promoter, and Renilla Luciferase controlled by the viral Thymidine kinase promoter. The ratio of Firefly to Renilla Luciferase activity is a specific measure of Hh pathway activity. This panel shows the ratio of Firefly:Renilla Luciferase activity in ShhLIGHT2 cells treated in serum-free medium with (1) untransfected serum-free HeLa cell supernatants (mock), (2) with Shh obtained from Shh-transfected HeLa cells grown in serum-free media (ShhN*). HeLa derived Shh + HDL, LDL or VLDL lipids. These lipids were added in DMSO at a concentration that mimicked that present in the serum. Error bars indicate standard deviation of 3 independent experiments.
   (b) Lipids derived from VLDL can destabilize Smo in the wing disc.
   (b'-b****) Wing discs dissected from either wild type (b') or LppRNAi (b*-b****) animals were incubated with liposomes consisting of DOPC (mock) or DOPC + lipids derived from HDL, LDL or VLDL, at concentrations corresponding to those present in the serum. After 2 hours at room temperature, discs were fixed and stained for Smo. In wild type mock-treated discs (b'), Smo is destabilized post-translationally by Patched in the anterior compartment. In mock-treated discs from Lpp knock-down animals (b*) Ptc-mediated Smo destabilization is inhibited. Incubating LppRNAi discs with lipids derived from VLDL (b****) but not HDL or LDL (b**, b***) reverses ectopic Smo accumulation in the anterior compartment. Scale bar = 10µM.
   (a) Quantification of Smo staining in mock-treated discs from wild type and LppRNAi animals, and LppRNAi discs incubated with lipids derived from HDL, LDL or VLDL. n>10 discs for each quantification.

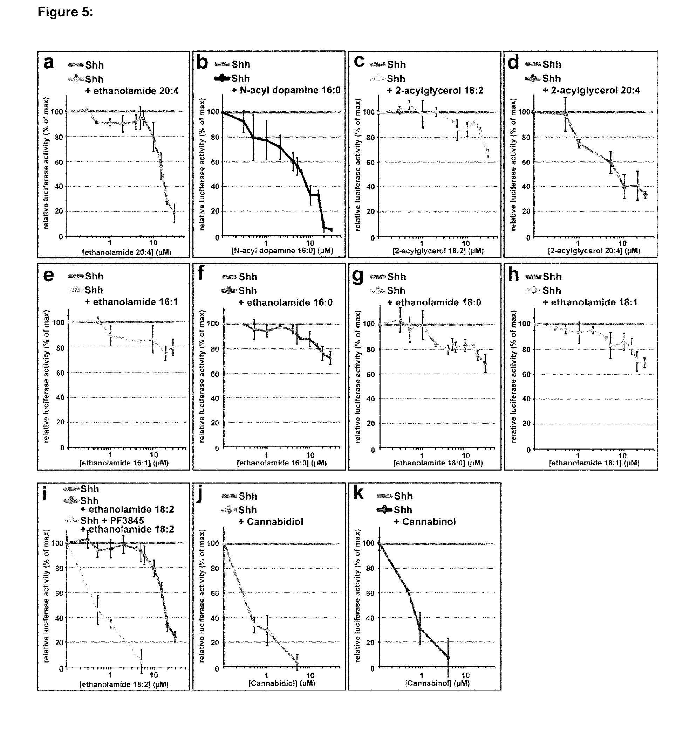

**Figure 5****: Specific endocannabinoids inhibit signaling in ShhLIGHT2 cells**
   (a-g) Effects of increasing concentrations of N-acylethanolamide (20:4) (a), N-acyl dopamine (16:0} (b), N-acylethanolamide (16:1) (c), N-acylethanolamide (16:0) (d), N-acylethanolamide (18:0) (e), N-acylethanolamide (18:1) (f), 2-acyl glycerol (18:2) (g) and 2-acyl glycerol (20:4) (h) on signaling by ShhN* in ShhLIGHT2 cells.
      For each experiment, the Firefly:Renilla ratio in cells not treated with Shh is defined as 0 to 0, and the fold increase in the Firefly:Renilla ratio obtained by addition of Shh is normalized to 100 (indicated by a green line). Fold stimulation by Shh in these experiments ranged from 3 to 5. Error bars indicate standard deviations of at least 5 independent experiments.
   (i) Effect of increasing concentrations of N-acylethanolamide (18:2) on signaling by Shh in ShhLIGHT2 cells in the absence and presence of a selective inhibitor of fatty acid amide hydrolase, PF-3845. The EC50 of N-acylethanolamide (18:2) is decreased over 10-fold by addition of PF-3485. Error bars indicate standard deviations of 3 independent experiments.
   (j,k) Effects of increasing concentrations of Cannabidiol and Cannabinol on signaling by Shh in ShhLIGHT2 cells. Error bars indicate standard deviations of 3 independent experiments.

The examples illustrate the invention:

### Example 1: Experimental Procedures

### Mammalian expression plasmids

cDNA encoding human Shh in pCMV-XL5 vector was purchased from OriGene (SC300021).

### Fly stocks

Oregon R flies, hs-flippase and adh-GAL4 are available from the Bloomington Stock Center. Transgenic line UAS<HcRed>dsLpp is described in Panáková, D., et al., Nature, 2005. 435(7038): p. 58-65.

### Induction of RNAi

LppRNAi was induced as described Panáková, D., et al., Nature, 2005. 435(7038): p. 58-65.

### Immunohistochemistry

Imaginal discs were fixed and stained as previously described (Marois et al., 2006). Antibodies were diluted as follows: anti-Ci 2A1 1:10 (Wang and Holmgren, Development, 126(22): p. 5097-106 (1999)), anti-Smo 1:50 (Lum et al., Mol Cell, 12(5): p. 1261-74 (2003)).

### Image analysis

All quantified immunostaining was performed on discs that were dissected, fixed, stained and imaged in parallel using the same microscope settings. To quantify Ci155 and Smo staining intensities, three apical sections 0.7 µm apart were projected using maximal intensity in ImageJ. For each image, two rectangles of 100 pixels parallel to the A/P axis by 351 pixels parallel to the D/V axis were selected and centered at the A/P boundary in ventral and dorsal compartments. Average pixel intensity was determined as a function of distance from A/P boundary using PlotProfile and plotted using Microsoft Excel. All A/P boundaries were determined according to anti-Ci155 coimmunostaining. To estimate the significance of changes in staining intensities in discs of different genotypes, we measured Smo or Ci staining intensity at the same distance from the A/P boundary in each disc and calculated p values using Excel.

### Lipoprotein isolation

VLDL, LDL and HDL were isolated from human serum (Sigma) essentially according to (Redgrave et al., Anal Biochem 65, 42-49 (1975)). Drosophila Lipophorin (Lpp) was isolated as described previously (Khaliullina et al., Development, 136(24): p. 4111-21 (2009)).

### Hemolymph isolation

Hemolymph was collected from punctured 3rd instar larvae in PBS, and centrifuged for 30 min at 1500 g, then for 30 min at 16 000 g. Stronger centrifugation pellets a large fraction of lipoproteins and hexamerins.

### Lipid extraction

Lipids were extracted from purified VLDL, LDL, HDL and Lpp particles by a two-step Bligh and Dyer method (Bligh and Dyer, Canadian Journal of Biochemistry and Physiology, 37(8): p. 911-7 (1959)). Total lipid concentration was measured according to (Marsh and Weinstein, Journal of lipid Research, 7(4): p. 574-576 (1966). Lipoprotein-derived lipids were applied to LIGHT2 cells and wing discs at concentrations found in hemolymph/ human serum.

### Drosophila wing disc assay

The assay was performed as previously described (Khaliullina et al., Development, 136(24): p. 4111-21 (2009)). Endocannabinoids were applied in DOPC vesicles at concentrations of up to 200µM.

### Preparation of saponification-resistant lipids

To deplete lipids destroyed by saponification, dried VLDL lipid extracts were incubated at 80°C with 2 ml of 0.3 M methanolic potassium hydroxide for one hour. After cooling, the solution was extracted by two washes with diethyl ether. The ether fractions, which contain saponification-resistant lipids, were combined and subjected to further analysis.

### Column chromatography

Extracts containing saponification-resistant lipids from VLDL were loaded onto a C18 column with following parameters: 25mm x 4,5mm, 5µM particle (Vydac, 218TP54). The extract was loaded in 60% methanol in H2O, washed with an isocratic step of 60% for 10min and eluted with an isocratic step of 80% methanol in H2O for 60min followed by a linear gradient from 80% to 100% methanol in H2O for 40min at the flow rate of 1ml/min. 40 fractions of 1,5ml were taken, dried down and subjected to the application in the LIGHT2 signaling assay.

### Mass spectrometric analysis

50 µl of each fraction was mixed with 65 µl of 13 mM ammonium acetate in iso-propanol. Prior to analyses, 20 µl of each sample were loaded into 96-well plate (Eppendorf, Hamburg), sealed with aluminum foil and centrifuged for 5 min at 4,000 rpm on a Multifuge 3S-R centrifuge from Heraeus DJB Labcare Ltd (Newport Pagnell, UK). Mass spectrometric analyses were performed on a Q Exactive (Thermo Fisher Scientific, Bremen) instrument. Infusion of samples was performed with a robotic nanoflow ion source TriVersa NanoMate (Advion BioSciences, lthaca NY) controlled by the Chipsoft 6.4 software. We used nanoflow chips with a 4.1 µm spraying nozzle diameter; ionization voltage was +/- 1.25 kV and gas back pressure 0.95 psi. FTMS in positive and negative ion mode was acquired with target mass resolution of Rm/z=200 =140 000 within m/z range of 100-1000. Precursors within m/z range of 200-500 were fragmented in a data-dependent acquisition mode with the target resolution of Rm/z=200 =17 000.

### Preparation of non-lipoprotein-associated Shh

HeLa cells were passaged in DMEM with 10% fetal bovine serum (FBS, Gibco), 50 U/ml penicillin and 50µg/ml streptomycin (Gibco).
To prepare non-lipoprotein-associated Shh, HeLa cells were transfected with plasmids encoding human Shh using polyethylenimine (Polysciences) in OptiMem (Invitrogen), then switched Shh-transfected HeLa cells to serum-free media (DMEM +1% Insulin-Transferrin-Selenium mixture (ITS-X, Gibco) 4 h post transfection. After 48 h, conditioned media were centrifuged at 1000 g for 20 min and concentrated using Amicon Ultra -10K (Millipore) for use in the signaling assay. Identically treated media from nontransfected HeLa cells were used as controls.

### ShhLIGHT2 signaling assay

Shh-LIGHT2 cells (Taipale et al., 2000) were maintained in DMEM + 10% FBS, 150 µg/ml zeocin (Invitrogen) and 400 µg/ml G418 (Invitrogen). 24 h prior to assay, cells were plated at 105/well in 96-well plates. Cells were then switched to a serum-free medium consisting of DMEM + 1% ITS-X, and supplemented with combinations of Shh and different lipids. Lipids were added in DMSO.
Luciferase activity was assayed in cell lysates after 24 h, as instructed by the manufacturer (Dual Glo Luciferase Assay, Promega).

### Example 2:

Both *Drosophila* and mammalian assays were used to assess pathway inhibition by lipoprotein-derived lipids: their ability to inhibit signaling by non-lipoprotein-associated Shh in mammalian ShhLIGHT2 cells (Sasaki, H., et al., Development, 1997. 124(7): p. 1313-22) was tested; it was also asked whether they could reverse the effects of Lpp knock-down in explanted *Drosophila* wing discs. When lipoprotein lipids are depleted by Lpp RNAi, Smo accumulates ectopically throughout the anterior compartment of the wing disc. Smo blocks processing of the Gli family transcription factor Cubitus interruptus (Ci) to its repressor form, causing ectopic accumulation of full-length Ci₁₅₅. Both Smo and Ci₁₅₅ accumulation are reversed by the addition of protein-free liposomes containing Lpp lipids to explanted Lpp RNAi discs (fig.1a and Khaliullina, H., et al., Development, 136(24): p. 4111-21 (2009)).

Since initial experiments showed that VLDL carried lipids that were active both in *Drosophila* wing disc assays and in ShhLIGHT2 cells, the search was focused on VLDL. Lipids extracted from both Lpp and VLDL are still active after saponification in both mammalian and *Drosophila* assays (fig.1), suggesting that inhibitory lipids are conserved, and that they are neither phospholipids nor di- or triacylglycerols.

7-dehydrocholesterol and its derivative Vitamin D3 inhibit signaling by vertebrate Smo (Bijlsma, M.F., et al., PLoS biology, 2006. 4(8): p. e232). Because LppRNAi depletes *Drosophila* discs of sterols (Khaliullina, H., et al., Development, 136(24): p. 4111-21 (2009)), it was considered whether depletion of these molecules might cause Smo accumulation in LppRNAi discs. However, although 7-dehydrocholesterol and vitamin D3 inhibited signaling in ShhLIGHT2 cells and (Bijlsma, M.F., et al., PLoS biology, 4(8): p. e232 (2006)), neither reversed Smo accumulation in LppRNAi discs. Indeed, no sterol species that were tested did so. Thus, the conserved inhibitory lipids are unlikely to be sterol derivatives. Since sphingolipids also resist saponification, different species of ceramides, phytoceramides, sphingosines, phytosphingosines and sphingosine-1-phosphate were assayed in both ShhLIGHT2 cells and in LppRNAi discs. None of these lipids destabilized Smo in discs or repressed Shh-mediated signaling in ShhLIGHT2 cells.

Therefore, to identify conserved inhibitory lipids, saponified lipid extracts of VLDL were fractionated by reverse phase (C18) HPLC, assaying column fractions for their ability to inhibit Shh signaling in ShhLIGHT2 cells. This revealed several clusters of column fractions with inhibitory activity (fig.2a, fractions 3 to 6, 10 to 12, 25 to 32), and one region with stimulatory activity (fig.2a, fractions 1 and 2).

MS/MS and FTMS was used to identify lipids with known signaling functions in the different column fractions. Fractions with inhibitory/stimulatory activities do not coincide with peaks of 20-OH cholesterol, and Vitamin D3 is not detected in VLDL fractions. Neither do the peaks of activity overlap with fractions containing sphingolipids. However, specific endocannabinoids cofractionate with each region of activity. Endocannabinoids comprise a family of endogenous compounds that consist of fatty acids linked to a variety of polar head groups. They were originally defined as ligands for the G protein-coupled cannabinoid receptors CB1 and CB2, although additional endocannabinoid receptors are emerging. These include GPCR's, nuclear hormone receptors and ion channels (Pertwee, R.G., et al., Pharmacological reviews, 62(4): p. 588-631 (2010)). Fractions with repressive activity towards the Hh pathway correspond to peaks of N-acylethanolamides, 2-glyceryl ethers and N-acyl dopamines. Although VLDL lipids had been saponified, also detected were residual amounts of 2-acyl glycerols in fractions with repressive activity. Fractions with stimulatory activity correspond to peaks of N-acyl serines. Thus, VLDL particles carry endocannabinoids that are Shh pathway inhibitors and activators.

To broadly assess the effects of endocannabinoids on the Hh pathway, commercially available compounds were obtained in pure form and assayed them in ShhLIGHT2 cells. Specific species were tested from each endocannabinoid class found in activating or repressive column fractions. Members of each class potently modulated Hh signaling (fig.2b-e). N-acyl serine stimulated signaling in ShhLIGHT2 cells (fig.2b). In contrast, N-acyl dopamines, N-acylethanolamides, and 2-glyceryl ethers were repressive (figs.2c-e). Taken together, these data indicate that specific endocannabinoid classes account for the repressive activities found in VLDL particles, and that their activity masks the influence of additional, stimulatory endocannabinoids - the N-acyl serines.

The concentration of exogenously added free endocannabinoids required to influence Hh signaling (1-10 µM) is high relative to normal ligand/receptor binding affinities. However catabolism of endocannabinoids might reduce their effective intracellular concentration. It was therefore asked whether inhibiting fatty acid amide hydrolase (FAAH) might allow endocannabinoids to act at lower concentrations. Indeed, PF-3845, a selective FAAH inhibitor, reduces the EC₅₀ of N-acylethanolamide (18:2) to 500nM. Thus, endocannabinoids added in soluble form are probably catabolized by FAAH. Since cells are normally exposed to endocannabinoids carried in VLDL, lipoprotein vehicles may target them to a protected subcellular compartment. To test this idea, it was asked whether loading onto lipoproteins might reduce the amount of endocannabinoid required to inhibit Hh signaling. N-acylethanolamide (18:2) is active at more than 10-fold lower concentrations when preincubated with lipoproteins (fig.2e). Thus, lipoprotein vehicles may allow signaling lipids to enter protected compartments with access to Hh pathway components.

The effects of endocannabinoids on cannabinoid and vanilloid receptors are mimicked by molecules found in *Cannabis.* To ask whether Cannabis-derived cannabinol or cannabidiol might also influence Hh signaling, their effects were assayed in ShhLIGHT2 cells. Both inhibited pathway activity with an EC₅₀ less than 1 µM. Thus, these compounds exert some of their physiological effects by repressing Hh signaling.

Since previous experiments suggested that lipoprotein inhibitory lipids were conserved between *Drosophila* and humans (fig.1 and Palm et al., submitted), it was wondered whether *Drosophila* Lpp might also carry endocannabinoids. To investigate this, we analyzed hemolymph from wild type and LppRNAi animals by MS/MS. Two endocannabinoids, N-acylethanolamide (16:0) and 2-glyceryl ether (14:1), were detected whose levels were lowered by Lpp knock-down (fig.3a,b). Thus, *Drosophila* Lpp particles carry a subset of the endocannabinoids present in VLDL.

To ask whether endocannabinoids influenced the *Drosophila* Hedgehog pathway, different endocannabinoid classes and species were added to wild type and LppRNAi wing discs and measured their effects on Smo and Ci₁₅₅ accumulation. We observed no activity of N-acyl serine (16:0), N-acyl dopamine (16:0) or 2-glyceryl ether (20:4) by these methods. In contrast, N-acylethanolamide (16:0) and 2-acyl glycerol (20:4) had strong negative effects on Hh signaling in *Drosophila.* N-acylethanolamide (16:0) completely reverses ectopic accumulation of Smo in the anterior compartment of LppRNAi discs, reducing Smo staining intensity to that observed in wild type discs (fig.3c). It also significantly reverses ectopic accumulation of Ci₁₅₅ (fig.3d). In contrast, Smo and Ci₁₅₅ levels in LppRNAi discs were not decreased by N-acylethanolamides (18:0), (18:1) or (20:4). Thus, inhibition of Hh signaling by N-acylethanolamides in *Drosophila* requires species with different fatty acid moieties than those active in mammalian ShhLIGHT2 cells. Taken together, these data suggest that N-acylethanolamide (16:0) contributes to the negative effects of Lpp on Hh signaling in the wing disc, and that its depletion in Lpp RNAi wing discs at least partially accounts for ectopic stabilization of Smo and Ci₁₅₅.

2-acyl glycerol (20:4) reverses Smo and Ci₁₅₅ accumulation in LppRNAi wing discs in both the anterior and posterior compartments, and reduces Ci₁₅₅ to less than wild type levels. Furthermore, 2-acyl glycerol (20:4) has similar effects even on wild type discs (fig.3e,f). The complete inactivity of the corresponding ether-linked molecule, 2-glyceryl ether (20:4), - suggests stringent structural requirements for its effects on the Hh pathway in *Drosophila.* Taken together, these data demonstrate a conserved function for N-acylethanolamides and 2-acyl glycerols in repressing the Hh pathway in mammals and in flies.

How might endocannabinoids influence Hh signaling? Endocannabinoids bind several mammalian receptors including the GPCR's CB1 and CB2 and the vanilloid receptor (TRPV). However, *Drosophila* do not have homologues of CB1 or CB2 (McPartland, J., et al., The Journal of comparative Neurology, 436(4): p. 423-9 (2001)), and mutations in the TRPV channel components *inactive* and *nanchung* do not produce Hh signaling phenotypes. Since endocannabinoids affect both *Drosophila* and mammalian Hh signaling, it is unlikely that they act through these receptors.

## Claims

1. A pharmaceutical composition comprising one or more endocannabinoids, cannabinoids and/or modified versions thereof complexed with one or more lipoproteins, wherein said one or more endocannabinoids are selected from N-acylethanolamides, N-acyl dopamines, 2-acyl glycerols and 2-glyceryl ethers and wherein said one or more cannabinoids are selected from cannabinols, cannabidiols and tetrahydrocannabinols.

2. A pharmaceutical composition comprising one or more endocannabinoids, cannabinoids and/or modified versions thereof, wherein said one or more endocannabinoids are selected from N-acylethanolamides with the exception of N-acylethanolamide (16:0), N-acylethanolamide (18:1) and N-acylethanolamide (20:4); N-acyl dopamines with the exception of N-acyl dopamine (16:0); 2-acyl glycerols with the exception of 2-acyl glycerol (20:4); and 2-glyceryl ethers with the exception of 2-glyceryl ether (20:4); and wherein said one or more cannabinoids are selected from cannabinols with the exception of cannabinol; cannabidiols with the exception of cannabidiol; and tetrahydrocannabinols with the exception of tetrahydrocannabinol.

3. The pharmaceutical composition according to claim 2, wherein said one or more endocannabinoids, cannabinoids or modified versions thereof are complexed with a lipoprotein.

4. The pharmaceutical composition according to any one of claims 1 or 3, wherein the lipoprotein is a very low density lipoprotein (VLDL) and/or high density lipoprotein (HDL).

5. The pharmaceutical composition according to any one of claims 1 to 4, wherein
(a) in case the pharmaceutical composition comprises one or more endocannabinoids selected from N-acylethanolamides, N-acyl dopamines and/or modified versions thereof said pharmaceutical composition further comprises one or more fatty acid amide hydrolase (FAAH) inhibitors; and/or
(b) in case the pharmaceutical composition comprises one or more 2-acyl glycerols and/or modified versions thereof said pharmaceutical composition further comprises one or more monoacylglycerol lipase inhibitors.

6. The pharmaceutical composition according to any one of claims 1 to 5, wherein said pharmaceutical composition further comprises one or more hedgehog signaling pathway inhibitors.

7. The pharmaceutical composition according to claim 5, wherein the fatty acid amide hydrolase (FAAH) inhibitor is selected from the group consisting of PF-3845, URB597, URB754 and JZL195.

8. The pharmaceutical composition according to any one of claims 1, and 3 to 7, wherein said pharmaceutical composition further comprises a GPI-linked protein, a sterol-linked protein and/or a palmitoylated protein.

9. The pharmaceutical composition according to any one of claims 1 and 4 to 8, wherein the one or more endocannabinoids are selected from the group consisting of N-acylethanolamide (18:2), N-acylethanolamide (20:4), 2-glyceryl ether (16:0), 2-glyceryl ether (16:1), 2-glyceryl ether (18:1), 2-glyceryl ether (20:4), N-acyl dopamine (16:0), N-acyl dopamine (18:0) and/or modified versions thereof; and wherein the one or more cannabinoids are selected from the group consisting of cannabinol, cannabidiol and/or modified versions thereof.

10. A pharmaceutical composition according to any one of claims 1 and 3 to 9 for use in the treatment of a tumour in a mammal.

11. The pharmaceutical composition for use according to claim 10, wherein the tumour is a hedgehog signaling-dependent tumour.

12. A pharmaceutical composition according to any one of claims 2 to 9 for use in the treatment of a tumour in a mammal, wherein said tumour is a hedgehog signaling-dependent tumour.

13. The pharmaceutical composition for use according to claim 11 or 12, wherein the hedgehog signaling-dependent tumour is selected from the group consisting of ovarian cancer, transitional cell carcinoma of the bladder, basal cell carcinoma, medulloblastoma, chronic myeloid leukemia, esophageal cancer and multiple myeloma.

14. A method for modulating the activity of the hedgehog signaling pathway in a mammalian cell, the method comprising the step of:
contacting said cell with one or more endocannabinoids selected from
(a) the group consisting of N-acylethanolamides, N-acyl dopamines, 2-acyl-glycerols, 2-glyceryl-ethers and/or modified versions thereof; or
(b) the group consisting of N-acyl serines and/or modified versions thereof; and/or contacting said cell with one or more cannabinoids selected from cannabinols, cannabidiols,
tetrahydrocannabinols and/or modified versions thereof,
thereby modulating the activity of the hedgehog signaling pathway in said mammalian cell.

15. Use of one or more endocannabinoids selected from
(a) the group consisting of N-acylethanolamides, N-acyl dopamines, 2-acyl-glycerols, 2-glyceryl-ethers and/or modified versions thereof;
(b) the group consisting of N-acyl serines and/or modified versions thereof; and/or cannabinoids selected from the group consisting of cannabinols, cannabidiols, tetrahydrocannabinols and/or modified versions thereof
in the differentiation of embryonic stem cells, progenitor cells and/or induced stem cells.
